# EUROPEAN PATENT APPLICATION

(11) **EP 4 063 494 A1**
(43) Date of publication of application: **28.09.2022**
(21) Application number: 21165236.7
(22) Date of filing: 26.03.2021
(51) Int. Cl.: C12N 5/0787, C12N 5/10, G01N 33/48

(54) **ASSAY**

(71) Applicant: Universität Bern, 3012 Bern (CH)
(72) Inventor: The designation of the inventor has not yet been filed
(74) Representative: HGF

(57) **Abstract**

A method for producing non-human conditionally immortalized mast cell progenitors comprises: introducing a nucleic acid molecule comprising an inducible homeobox gene into myeloid progenitor cells, wherein said myeloid progenitor cells are derived from a non-human animal and are engineered to express a heterologous high-affinity IgE receptor alpha subunit (FcεRIα); and selecting for cells which contain the nucleic acid molecule. The non-human conditionally immortalized mast cell progenitors may be cultured to obtain differentiated mast cells. The mast cells find utility in assays for the determination of IgE mediated allergies.

## Description

This invention relates to assays for allergy testing. The invention further relates to cell lines and kit for use in said assays, and methods of producing the cell lines.

### BACKGROUND

Roughly one-third of the global population is suffering from allergic hypersensitivity disorders, according to recent estimations. For many patients allergies are associated with a marked reduction in physical and psychological well-being and lead to a significant loss in quality-of-life due to disease activity. To provide efficient and personalized treatment options, physicians are dependent on solid and reliable diagnostic tools.

Generally, allergy diagnosis is a complex and laborious multistep procedure. It involves the examination of the patient's medical history, serological determination of total and allergen specific immunoglobulin E (IgE) antibody levels and various in vivo allergen skin prick tests (SPT) or other in vivo allergen challenge protocols. Even though the determined levels of total and allergen specific IgE antibodies provide information about the atopic status of an individual, these values often poorly correlate with disease activity and clinical symptoms. Further, important parameters contributing to aggravation or suppression of allergic responses such as diversity and affinity of the allergen-specific IgE antibodies and the presence of allergen-specific IgG antibodies are frequently neglected in the interpretation of diagnostic laboratory results.

Occasionally, a functional basophil activation test (BAT) with whole blood samples of the patient is performed to determine reactivity against certain allergens. While such assays are useful as they provide important quantitative and functional information about the allergic status of an individual, they are hampered by the use of fresh whole blood that has to be processed within hours in specialized diagnostic laboratories. Immediate analysis of whole blood is associated with major logistical challenges as its storage is not possible due to instability of the biological material. More recently, diagnostic testing of allergies based on primary human blood-derived mast cells (i.e. MAT) has been suggested by independent groups. While the overall goal of this approach is appealing, the generation of the cells is laborious and requires extended culturing periods of more than two months. Despite these interesting recent developments, a convenient, safe, standardized and reliable diagnostic assay predicting functional reactivity against culprit allergens is still not available.

Allergic patients are often advised to undergo allergen-specific immunotherapy (AIT), which has been reported to be one of the few disease modifying interventions currently available for allergy treatment. In AIT increasing doses of allergen (up-dosing phase) are applied to the patient until a certain maintenance dose (depends on allergen) is reached. In subcutaneous AIT (SCIT) protocols patients receive monthly allergen injections over a duration of three to five years after an initial up-dosing phase. Various molecular and cellular mechanisms such as the induction of allergen-specific protective IgG have been described for AIT, however, it remains still unclear why some patients respond better to the treatment than others. In fact, only 13% of patients show sustained unresponsiveness to an allergen after one year of AIT completion as assessed in a recent peanut desensitization study. Currently, there is no suitable read-out system available to assess whether and when a patient is responding to the treatment. Therefore, physicians usually perform an in vivo allergen challenge test to determine the degree of unresponsiveness after AIT, which is unpleasant for the patient and risks inducing an allergic reaction in case the patient has not responded to the treatment.

### BRIEF SUMMARY OF THE DISCLOSURE

According to a first aspect of the invention, there is provided a method for producing non-human conditionally immortalized mast cell progenitors, the method comprising:
- introducing a nucleic acid molecule comprising an inducible homeobox gene into myeloid progenitor cells, wherein said myeloid progenitor cells are derived from a non-human animal and are engineered to express a heterologous high-affinity IgE receptor alpha subunit (FcεRIα); and
- selecting for cells which contain the nucleic acid molecule.

According to a second aspect of the invention, there is provided a method for preparing mast cells comprising culturing non-human conditionally immortalized mast cell progenitors which are engineered to express a heterologous high-affinity IgE receptor alpha subunit (FcεRIα), wherein the conditionally immortalized mast cell progenitors further comprise a homeobox gene, the expression of the homeobox gene being under the control of an inducer, and wherein the conditionally immortalized mast cell progenitors are cultured in the absence of the inducer.

The non-human conditionally immortalized mast cell progenitors used to prepare the mast cells may be those prepared using the method of the first aspect of the invention.

In a third aspect, the present invention provides a non-human conditionally immortalized mast cell progenitor comprising a homeobox gene, the expression of the homeobox gene being under the control of an inducer, wherein said cell expresses a heterologous high-affinity IgE receptor alpha subunit (FcεRIα).

The non-human conditionally immortalized mast cell progenitor may be obtainable by the method according to the first aspect of the invention.

In a fourth aspect of the invention, there is provided a composition comprising a population of non-human conditionally immortalized mast cell progenitors according to the third aspect of the invention. The composition may further comprise the inducer which controls the expression of the homeobox gene.

According to a fifth aspect of the invention, there is provided a non-human mast cell, wherein the mast cell comprises a homeobox gene, the expression of the homeobox gene being under the control of an inducer, and wherein the mast cell expresses a heterologous high-affinity IgE receptor alpha subunit (FcεRIα).

The mast cell may be obtainable by the method according to the second aspect of the invention.

According to a sixth aspect of the invention, there is provided a composition comprising a population of non-human mast cells according to the fifth aspect of the invention.

In a further aspect, the invention provides a method for determining whether a patient is allergic to an allergen and/or the severity of a patient's allergy to an allergen, the method comprising:
- incubating mast cells with a sample comprising patient antibodies;
- contacting the mast cells with the allergen; and
- detecting activation of the mast cells,
wherein the mast cells are non-human mast cells according to the fifth aspect of the invention.

In a further aspect, the invention provides a method for monitoring the effectiveness of a therapy that is being used, that may be used in the future, or that has previously been used to treat a patient allergic to an allergen, the method comprising:
- incubating mast cells with a first sample comprising patient antibodies;
- contacting the mast cells with the allergen;
- determining a first level of activation of the mast cells; and
- comparing the determined first level with a reference level,
wherein the mast cells are non-human mast cells according to the fifth aspect of the invention.

The therapy may be allergen-specific immunotherapy (AIT).

The method may be for monitoring the effectiveness of an anti-allergy therapeutic agent being administered to a patient in need thereof. The anti-allergy therapeutic agent may be an anti-lgE agent. In some embodiments, the anti-allergy therapeutic agent is an agent which induces protective IgG.

In another aspect of the invention, there is provided a method for determining the potency of an allergen preparation, the method comprising:
- incubating mast cells with IgE specific for the allergen;
- contacting the mast cells with a sample of the allergen preparation;
- determining a level of activation of the mast cells; and
- optionally, comparing the determined level of activation with a reference level,
wherein the mast cells are non-human mast cells according to the fifth aspect of the invention.

In a further aspect of the invention, there is provided a method for allergenicity screening of a food additive or drug candidate, the method comprising:
- incubating mast cells with a sample comprising subject antibodies;
- contacting the mast cells with the food additive or drug candidate; and
- detecting activation of the mast cells,
wherein the mast cells are non-human mast cells according to the fifth aspect of the invention.

According to a further aspect of the invention, there is provided a method for determining a serum IgE concentration of a patient, the method comprising:
- incubating mast cells with a sample comprising IgE from the patient; and
- determining the amount of IgE bound to the surface of the mast cells;
wherein the mast cells are non-human mast cells according to the fifth aspect of the invention.

In some embodiments, the method is for determining the total IgE concentration in the sample. In other embodiments, the method is for determining the concentration of an allergen-specific IgE in the sample.

The invention further provides a kit for allergy testing, the kit comprising:
- non-human mast cells according to the fifth aspect of the invention;
- a reagent for detecting activation of the mast cells.

### DETAILED DESCRIPTION

The invention will now be described by way of example and with reference to the accompanying Figures, in which:

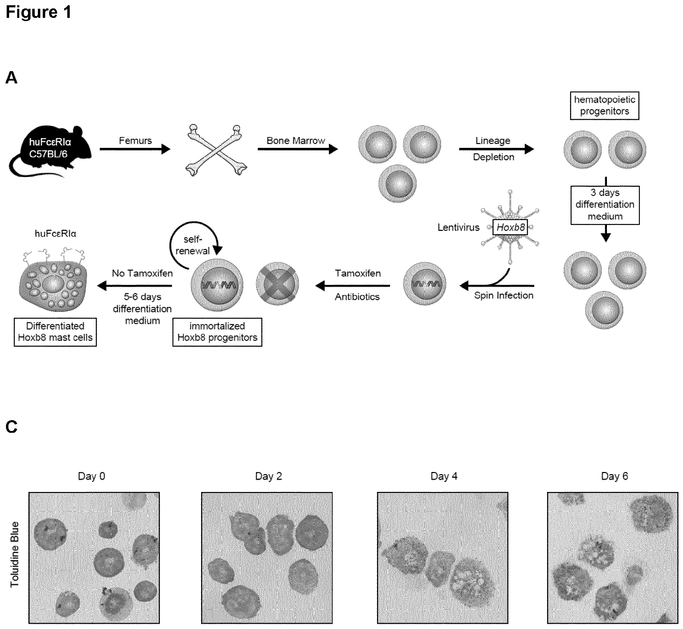

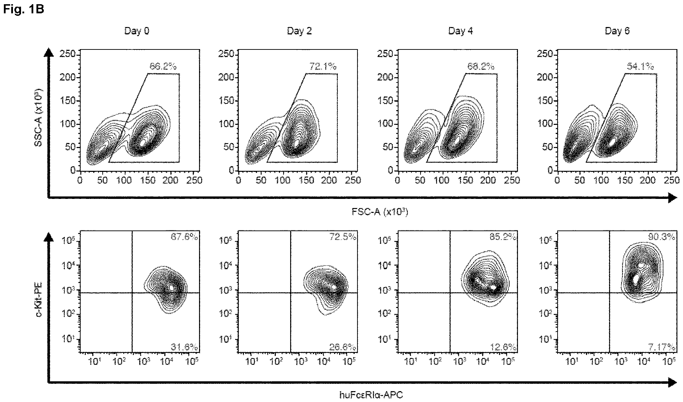

**Figure 1** shows the generation and differentiation of conditional immortalized Hoxb8 mast cell progenitors: **Fig.1A** is a schematic overview of progenitor line generation and differentiation; **Fig.1B** shows the flow cytometric assessment of the selected Hoxb8 mast cell progenitor line upon removal of the inducer 4-OTH. Cells are first gated on side- and forward-scatter (top row) and subsequently analysed for c-kit/huFcεRIα expression (bottom row); **Fig.1C** shows morphological analysis of the selected Hoxb8 mast cell progenitor line at day 0, 2, 4 and 6 of differentiation by toluidine staining;

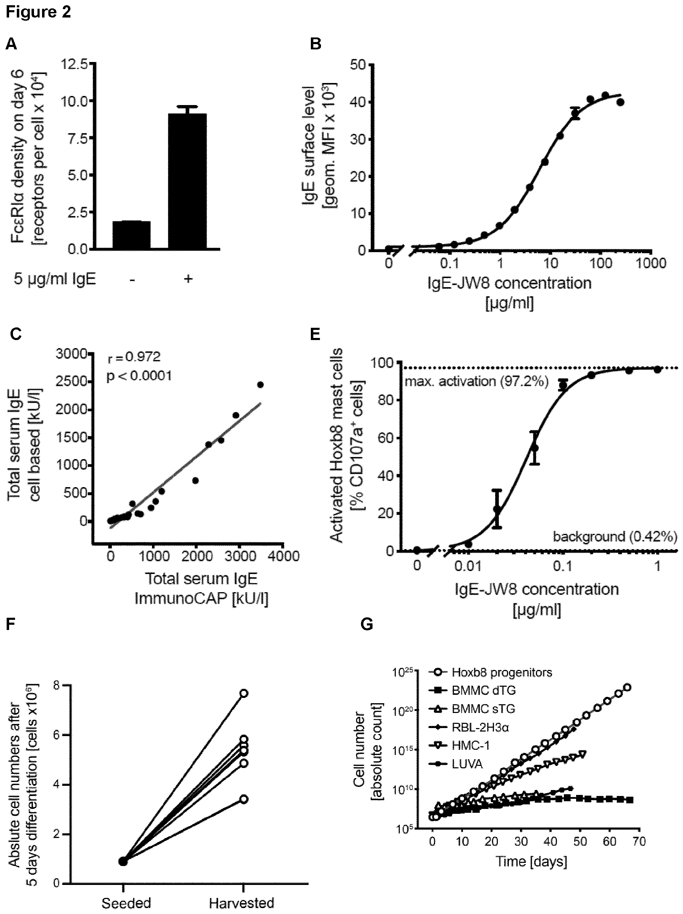

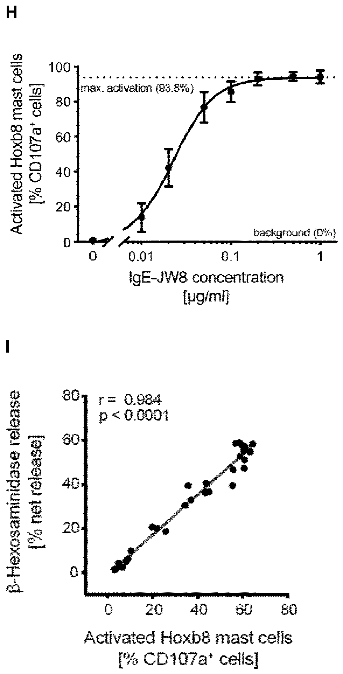

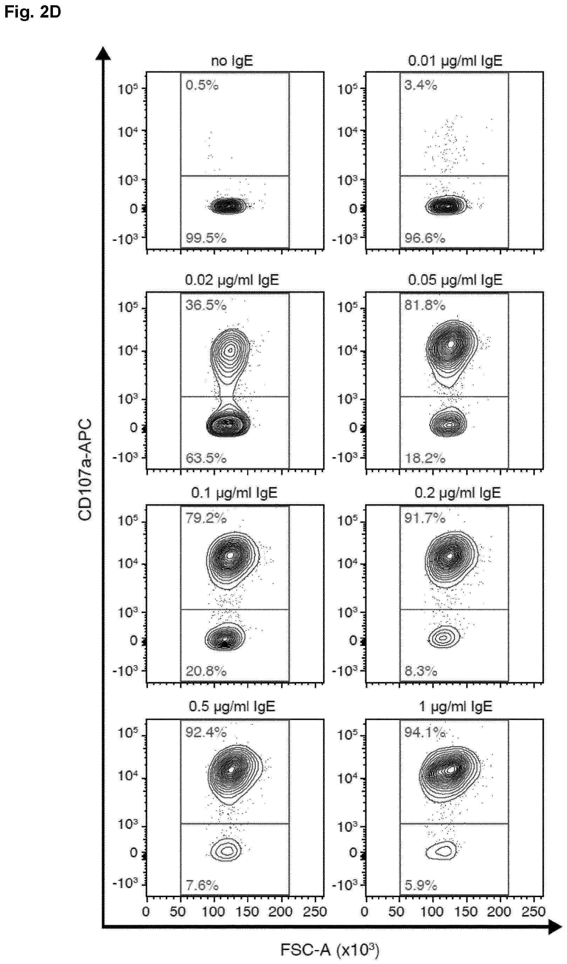

**Figure 2** shows the functional characterization of Hoxb8 mast cells after 6 days of differentiation: In **Fig.2A****,** the absolute number of huFcεRIα receptors per cell are shown in the absence or presence (overnight) of human recombinant IgE; **Fig.2B** shows dose-dependent binding of human recombinant IgE to differentiated Hoxb8 mast cells as assessed by flow cytometry; In **Fig.2C** the correlation between total serum IgE as determined on Hoxb8 mast cells or by singleplex immunoassay is shown for 25 allergic patient sera; **Fig. 2D** shows representative contour plots for antigen mediated activation of Hoxb8 mast cells in an IgE dose-dependent manner, as measured by flow cytometry; **Fig.2E** shows quantification of antigen mediated activation of Hoxb8 mast cells in an IgE dose-dependent manner; In **Fig.2F** absolute cell counts of seeded progenitor cells after 5 days of differentiation into harvested Hoxb8 mast cells are shown; In **Fig.2G** a comparison of cell growth for different allergic effector cells is shown over time; In **Fig.2H** quantification of antigen mediated activation of Hoxb8 mast cells in an IgE dose-dependent manner after 5 weeks of progenitor cell culture is depicted. A non-linear regression curve was fitted to measured data points. Data in Fig.2H are shown as mean ± SEM; In **Fig.2I** the correlation between released β-hexosaminidase and the cell surface activation marker CD107a is depicted for antigen activated Hoxb8 mast cells. Statistical analysis in C and I was performed using a standard linear regression model. Data in A, B and E are shown as mean ± SEM;

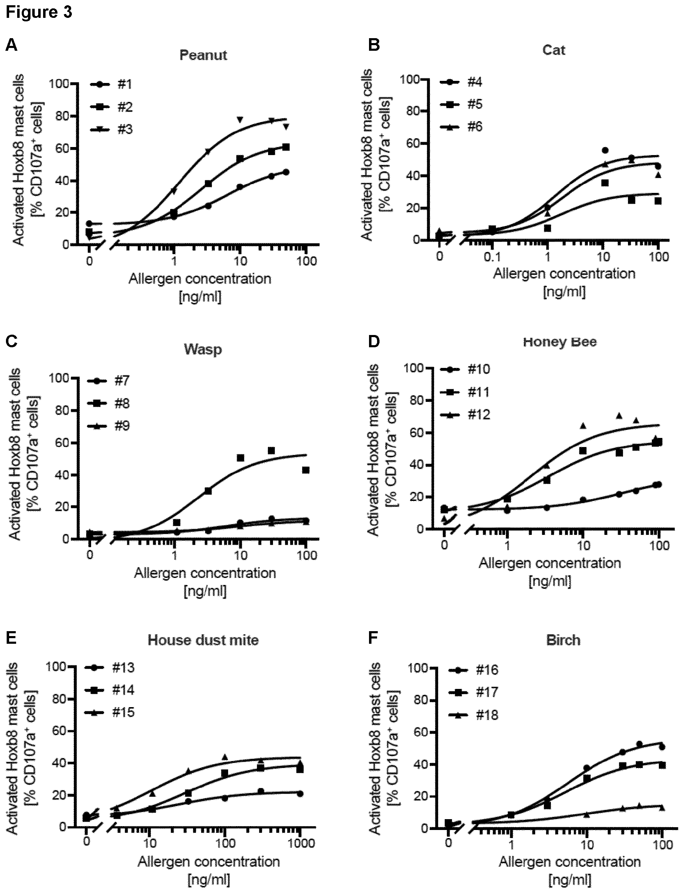

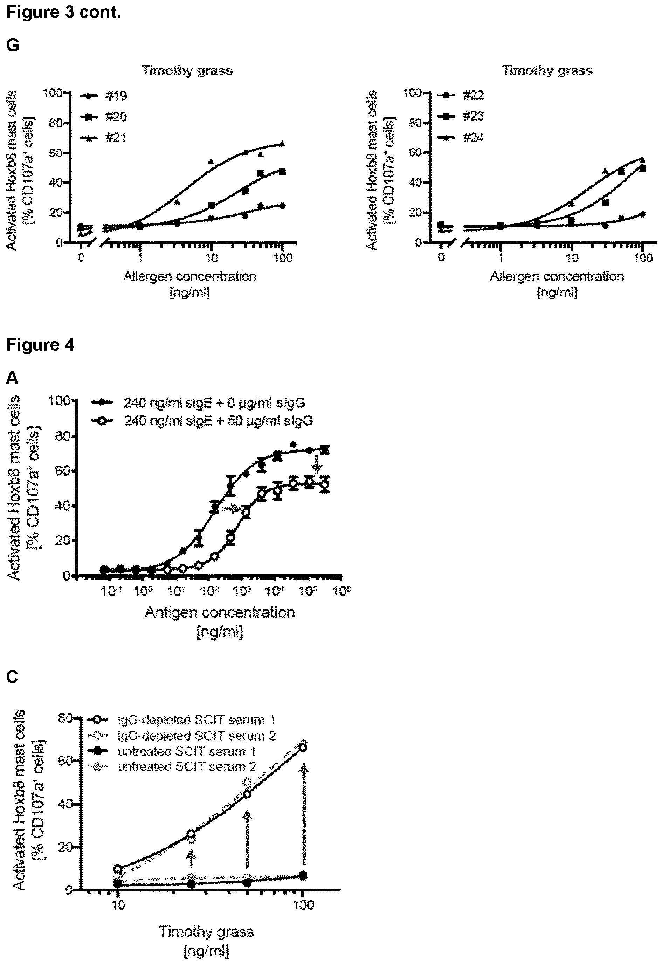

**Figure 3** Testing of allergic patient sera on Hoxb8 mast cells. Pre-defined sera of allergic patients were used to sensitize cells overnight. Dose-dependent activation as measured by flow cytometry is shown for different allergen sources: **Fig.3A** peanut extract; **Fig.3B** recombinant Fel d1; **Fig.3C** yellow jacket wasp venom; **Fig.3D** honey bee venom; **Fig.3E** house dust mite extract; **Fig.3F** common birch pollen extract; **Fig.3G** timothy grass. Non-linear regression curves were fitted to measured data points;

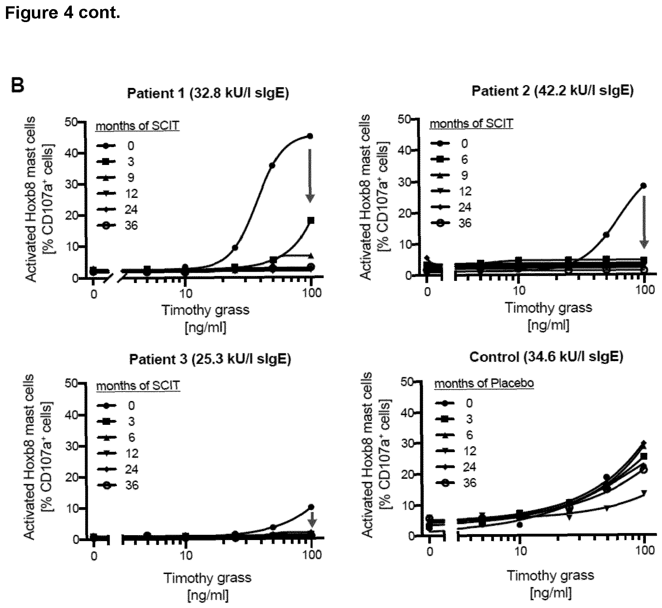

**Figure 4** Allergen-specific immunotherapy monitoring with Hoxb8 mast cells: **Fig. 4A****,** cells were sensitized overnight with an artificial serum containing either only human recombinant NIP-specific IgE (slgE) or a combination of human recombinant NIP-specific IgE (slgE) and IgG (slgG). Dose-dependent activation of the cells as measured by flow cytometry is shown; **Fig.4B****,** cells were sensitized overnight with sera from three timothy grass allergic patients that underwent AIT for at least 36 months and one serum of a patient undergoing placebo treatment. Dose-dependent activation of the cells as measured by flow cytometry is shown; **Fig.4C****,** cells were sensitized overnight with either untreated 483 or IgG-depleted sera from two timothy grass allergic patients (solid and dashed lines) at 12 months post AIT. Non-linear regression curves were fitted to measured data points. Arrows indicate shift of the curves;

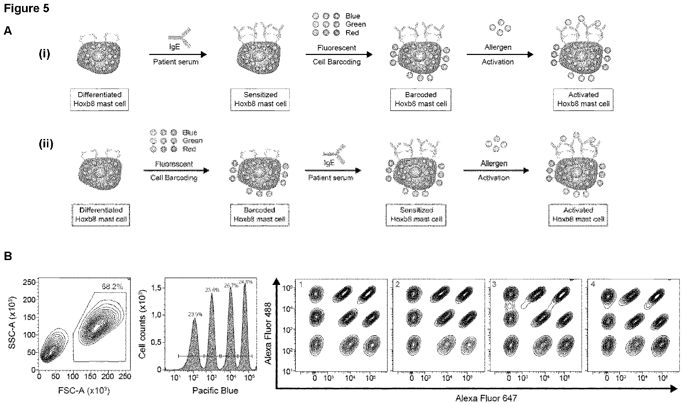

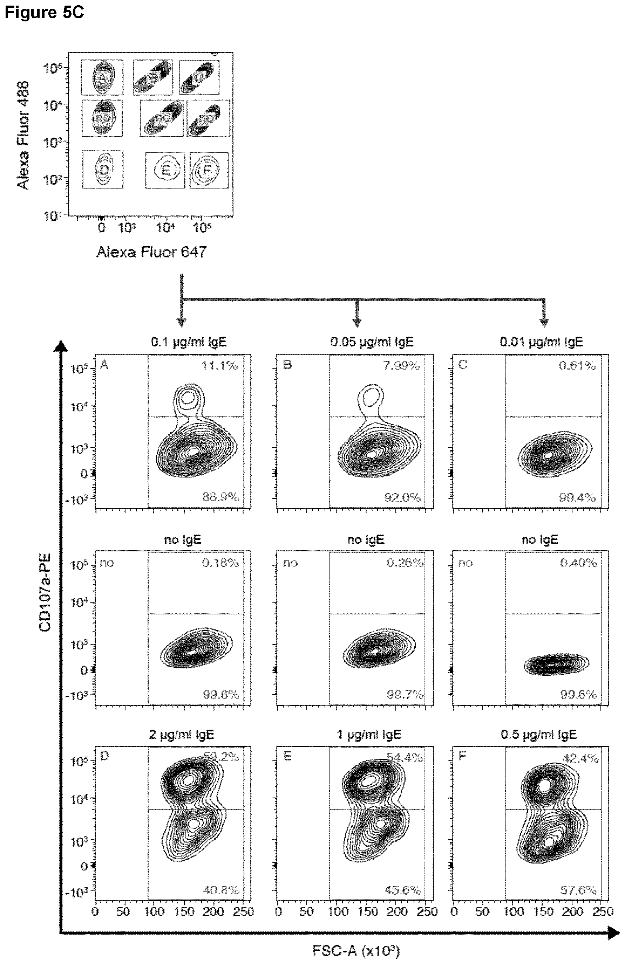

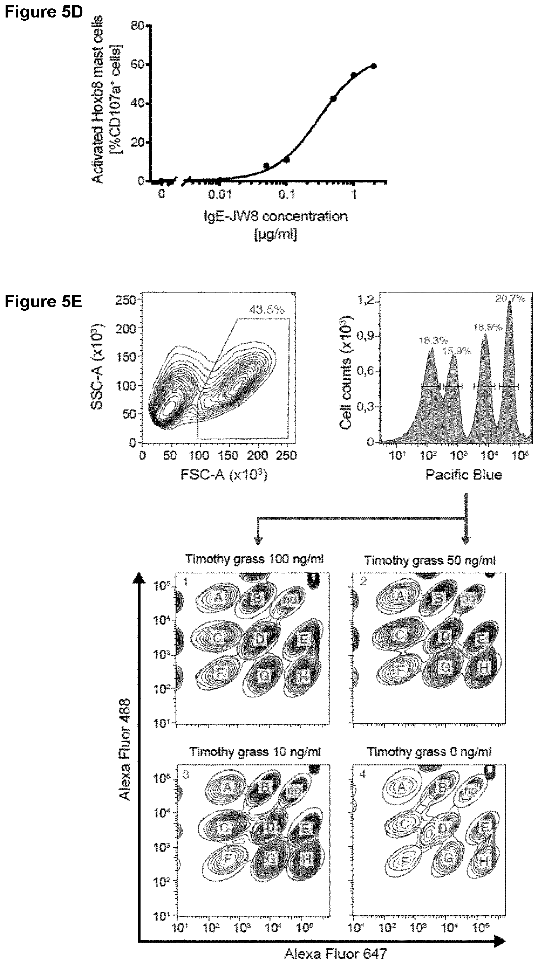

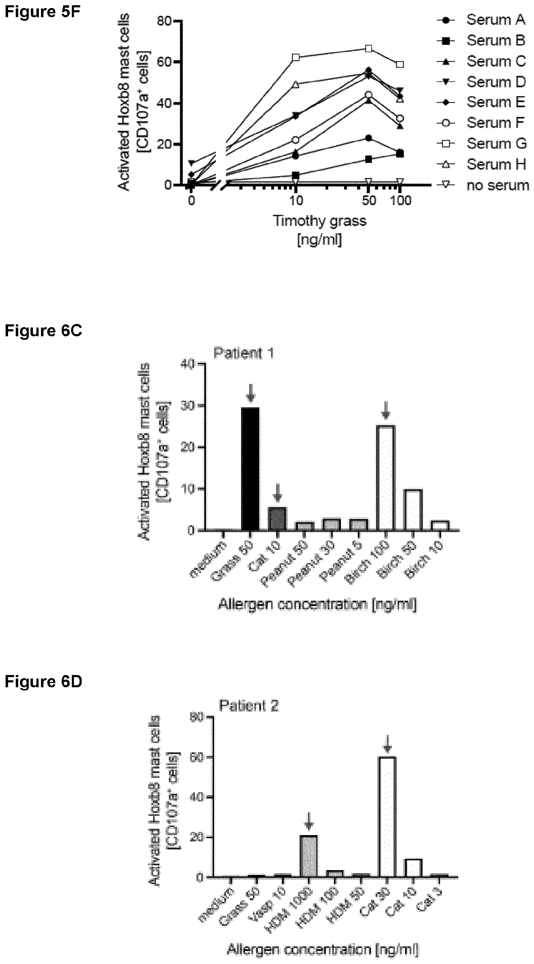

**Figure 5** High-throughput screening using cellular barcoding: **Fig.5A****,** schematic overview of alternative embodiments of the basic workflow are illustrated; **Fig.5B****,** the deconvolution gating strategy after acquisition of barcoded and pooled cells is depicted. Initially cells are gated on side- and forward-scatter. Then four different cell populations (1-4) are identified based on Pacific Blue labelling intensity. Each one of these four populations can be further subdivided into nine individual subpopulations based on the combination of Alexa Fluor 488 and Alexa Fluor 647 labelling intensities; **Fig.5C****,** individually barcoded cell populations (A-F) have been sensitized with a different concentration of human recombinant NIP-specific IgE. Cells were pooled and activated with NIP7BSA antigen. After acquisition the deconvolution analysis was performed to identify each individual cell subpopulation and to assess the activation status by quantifying CD107a as a cell surface activation marker; **Fig.5D****,** quantification of deconvoluted antigen mediated activation of Hoxb8 mast cells in an IgE dose-dependent manner for this high-throughput approach is shown. A non-linear regression curve was fitted to measured data points; **Fig.5E****,** Individually barcoded cell population (1A-4H) have been sensitized with sera from eight different timothy grass allergic patients. Cells were pooled in four separate tubes and activated with four concentrations of allergen. After pooled acquisition the deconvolution analysis was performed to identify each individual cell subpopulation and to assess the activation status by quantifying CD107a as a cell surface activation marker; **Fig.5F****,** quantification of deconvoluted dose-dependent allergen mediated activation of Hoxb8 mast cells for each individual patient sample is shown; and

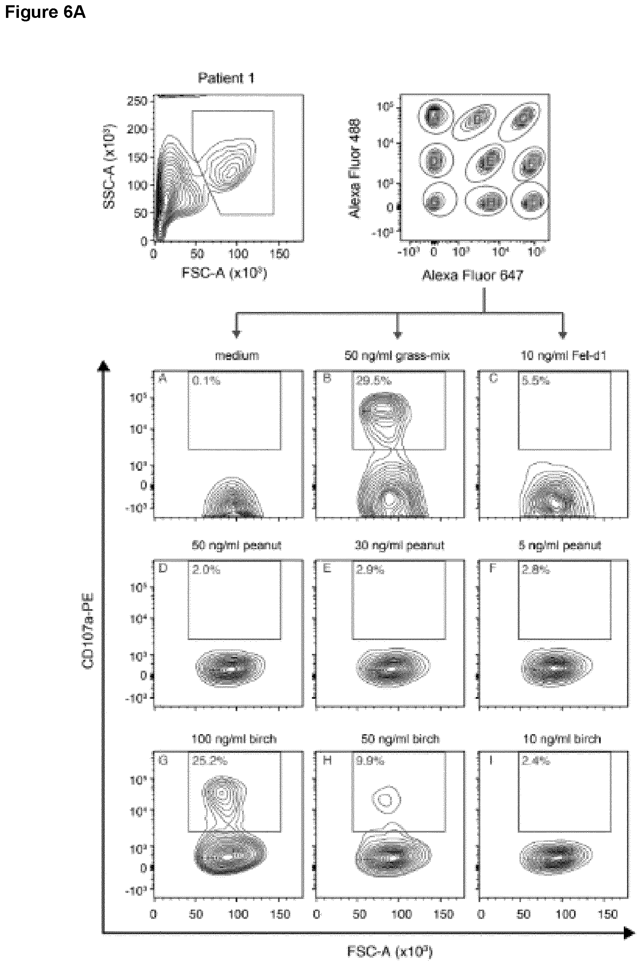

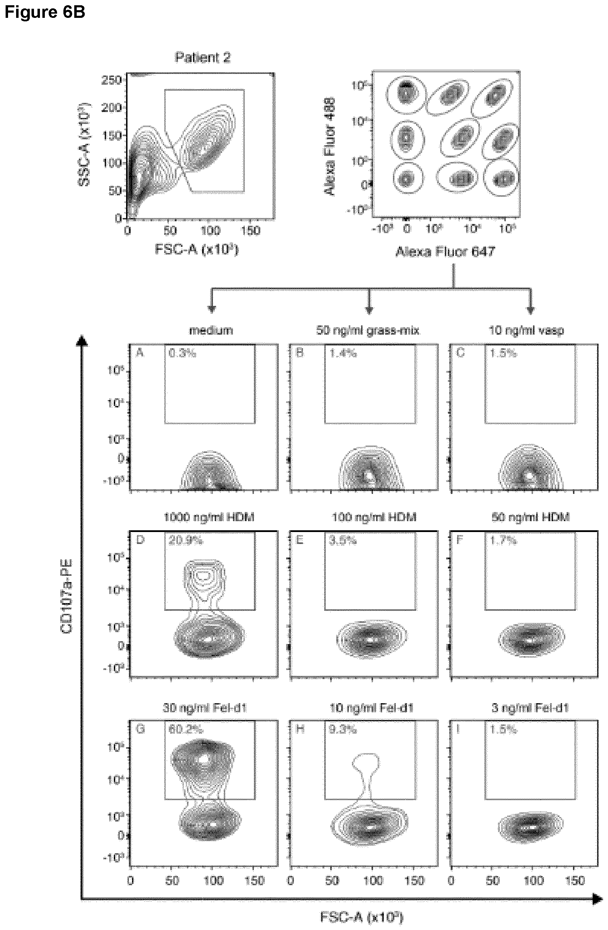

**Figure 6** High-throughput screening of multiple allergens using cellular barcoding. Individually barcoded cell populations have been sensitized with two sera (**Fig.6A** and **6B**) from polysensitized patients. The cells were stimulated with different recombinant allergens or allergen extracts. After pooled acquisition the deconvolution analysis was performed to identify each individual cell subpopulation and to assess the activation status by quantifying CD107a as a cell surface activation marker. **Fig.6C** and **6D****,** Quantification of deconvoluted dose-dependent allergen mediated activation of Hoxb8 mast cells for individual allergens with serum from patient 1 (**Fig.6C**) and patient 2 (**Fig.6D**) is shown. Arrows indicate the identified allergens that led to activation of the cells.

### Definitions

Unless otherwise stated, the following terms used in the specification and claims have the following meanings set out below.

"Myeloid progenitor cells" are a type of progenitor cells that differentiate into only a few cell types. Myeloid progenitor cells are precursors of red blood cells, platelets, granulocytes, monocyte-macrophages, dendritic cells, mast cells and osteoclasts.

"Mast cells" are cells which are present in virtually all vascularized tissues of adult mammals. Mast cells express on their surface the high-affinity receptor for IgE (FcεRI), which can be activated by IgE and specific antigens to release mediators such as histamine, leukotrienes, prostaglandins, serine proteases, and various cytokines, chemokines and growth factors. As such, mast cells are critical effector cells of IgE-associated allergic disorders. Mature mast cells are c-kit⁺ and FcεRI^{*}.

It has been found that mast cells do not mature before leaving the bone marrow, but circulate through the vascular system as immature mast cell progenitors. Thus, mast cells progenitors are precursors of mature mast cells which, *in vivo,* differentiate into mature mast cells under the influence of growth factors. In the context of the present invention, a "mast cell progenitor" is a cell which is capable of differentiating into a mature mast cell under certain conditions.

The phrase "high-affinity IgE receptor (FcεRI)" refers to the receptor for the Fc region of immunoglobulin E (IgE), an antibody isotype involved in the allergic response. FcεRI is a tetrameric receptor complex that binds Fc portion of the ε heavy chain of IgE. It consists of four polypeptide chains: an extracellular alpha chain (FcεRIα), a beta chain (FcεRIβ), and two gamma chains (FcεRIγ). The extracellular binding domain of the α-chain binds with high affinity to the Fc region of IgE, whereas the other chains are responsible for the transduction of initial cross-linking signals into the cell.

As used herein, the term "antibody" will be understood to include all antibodies and antigen binding fragments thereof, including whole antibodies, dimeric, trimeric and multimeric antibodies; bispecific antibodies; chimeric antibodies; recombinant and engineered antibodies, and fragments thereof. The term "antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lambda) bodies (scFv-CL fusions); Bispecific T-cell Engager (BiTE) (scFv-scFv tandems to attract T cells); dual variable domain (DVD)-Ig (bispecific format); small immunoprotein (SIP) (kind of minibody); SMIP ("small modular immunopharmaceutical" scFv-Fc dimer; DART (ds-stabilized diabody "Dual Affinity ReTargeting"); small antibody mimetics comprising one or more CDRs and the like.

### Production of mast cell progenitors

According to a first aspect of the invention, there is provided a method for producing non-human conditionally immortalized mast cell progenitors, the method comprising:
- introducing a nucleic acid molecule comprising an inducible homeobox gene into myeloid progenitor cells, wherein said myeloid progenitor cells are derived from a non-human animal and are engineered to express a heterologous high-affinity IgE receptor alpha subunit (FcεRIα); and
- selecting for cells which contain the nucleic acid molecule.

By "heterologous high-affinity IgE receptor alpha subunit (FcεRIα)" it will be understood that the gene encoding FcεRIα gene is derived from a different species to the non-human animal from which the myeloid progenitor cells are derived. In other words, the non-human animal is transgenic for the FcεRIα gene. In some embodiments, the FcεRIα gene is derived from the species for which the mast cells are to be used in the diagnostic and monitoring assays described herein. In some embodiments, the FcεRIα gene is human FcεRIα (accession no. NM_002001.4; Gene ID: 2005). For example, the myeloid progenitor cells may be mouse cells, and the FcεRIα may be human FcεRIα. Mice which are transgenic for human FcεRIα and which have the murine FcεRIα gene knocked out are described by Dombromovicz et al., J. Immunol. 1996, 15; 157(4): 1645-51, and can be obtained from the Jackson Laboratory (Strain B6.Cg-*Fcer1a^{tm1Knt}* Tg(FCER1A)1Bhk/J; Stock no. 010506).

The homeobox gene may be selected from HoxB8, HoxA9, Lhx2 (LH2) and TLX1 (Hox11). Lhx2 (LH2) and TLX1 (Hox11) have been shown to have the potential to immortalize multipotent haematopoietic progenitors (Pinto et al., EMBO J. 1998 Oct 1;17(19):5744-56; Zhang et al., Oncogene 1999 Apr 1;18(13):2273-9). In some embodiments, the homeobox gene is HoxB8. Hox genes of mammalian origin are well-known in the art. In some embodiments, the HoxB8 gene is mouse HoxB8. The mouse HoxB8 gene is available under GenBank accession no. NM_010461 (Gene ID: 15416) (encoding mouse HoxB8 protein accession No. NP_034591).

The homeobox gene is comprised within the nucleic acid molecule. Introduction of the homeobox gene into the myeloid progenitor cells can be achieved using any conventional recombinant technology for introducing nucleic acids into host cells including, but not limited to, transduction, transfection or electroporation. In some embodiments, the nucleic acid molecule may be any type of molecule which is suitable for transfection, such as a vector (e.g. a cosmid, plasmid or viral vector).

In some embodiments the nucleic acid molecule is a viral vector. Suitable viral vectors for the introduction of heterologous genes into cells (e.g. mammalian cells) will be known to the skilled person and include a herpes simplex viral vector, an adenoviral vector, an adeno-associated viral vector (AAV), or a retroviral vector, for example but not limited to, an HIV retroviral vector, a lentivirus, a VL 30 vector, a MSCV retroviral vector, or a Harvey Murine Sarcoma Vector. In some embodiments, the viral vector is a lentiviral particle.

Methods for subcloning the mouse Hoxb8 gene into a lentiviral system and generation of viral particles has been described by Salmanidis et al., Cell Death Differ 2013; 20:1370-80, and Gurzeler et al., Allergy 2013;68:604-13.

Methods for the introduction of a viral vector into host cells (i.e. transduction) will be known to those skilled in the art. In some embodiments, cells may be transfected by spin infection.

Cells into which the nucleic acid molecule has been successfully introduced (e.g. transduced cells) can be selected for by applying a selective pressure to the myeloid progenitor cells such that only those which contain the nucleic acid molecule survive. For example, the nucleic acid molecule may additionally comprise a gene conferring resistance to an antibiotic, in which case cells containing the nucleic acid molecule can be selected for by culturing the cells in the presence of the antibiotic (e.g. by culturing the cells in a culture medium comprising the antibiotic). Performing antibiotic selection enables elimination of cells which do not contain the nucleic acid molecule, resulting in a more homogenous (but still polyclonal) cell population.

In some embodiments, the cells are cultured in a culture medium comprising the antibiotic. Suitable antibiotics include, but are not limited to, puromycin and blasticidin. In some embodiments, the antibiotic is added to the culture medium 1, 2, 3, 4 or 5 days, preferably 2-4 days, after the introduction of the nucleic acid molecule (e.g. after transduction). The presence of the antibiotic in the culture medium may be maintained until outgrowth of the surviving cells (i.e. the transduced cells).

The mast cell progenitors produced by the method are conditionally immortalized. By "immortalized" it will be understood that the cells are capable of proliferating indefinitely. As such, the cells can be maintained in culture for long periods of time.

By "conditionally immortalized", it will be understood that the homeobox gene is not constitutively expressed but instead expression of the gene is controlled by an exogenous agent. As will be known by the skilled person, conditional expression of a gene can be achieved, for example, by inserting the gene downstream of an inducible promoter such that the gene is expressed the presence of the inducer. Any suitable inducible expression system may be used to control expression of the homeobox gene, such as the "Tet-on" or "Tet off" system.

Thus, in some embodiments, expression of the homeobox gene is controlled by an inducer. Myeloid progenitor cells which contain the nucleic acid molecule may be cultured in the presence of the inducer. For example, the culture medium may comprise the inducer. This causes expression of the homeobox gene, thereby immortalizing the cells. In some embodiments, the inducer is 4-hydroxytamoxifen (4-OHT). This inducer may be used with the expression system pF-5xUAS-gene_of_interest-GEV16.

In some embodiments, the culture medium used to culture the cells containing the nucleic acid molecule comprises interleukin-3 (IL-3).

Thus, following the introduction of the nucleic acid molecule into the myeloid progenitor cells, the cells may be cultured in culture medium comprising IL-3 and the inducer (e.g. 4-OHT). An antibiotic may be added to the culture medium (e.g. after 1-5 days, preferably after 2-4 days) to select for transduced cells, as described above.

Non-human conditionally immortalized mast cell progenitors obtained by the method described above may then be tested for their growth, viability and/or functional performance properties, in order to select the best performing cell lines.

In some embodiments, the method further comprises carrying out a single cell dilution of the conditionally immortalized mast cell progenitors, followed by clonal expansion so as to obtain a monoclonal conditionally immortalized mast cell progenitor cell line.

The myeloid progenitor cells which are used to prepare the conditionally immortalized mast cell progenitors may be derived from a non-human animal by:
- providing whole bone marrow previously obtained from the animal;
- enriching the bone marrow for hematopoietic progenitor cells; and
- culturing the hematopoietic progenitor cells in the presence of IL-3.

In some embodiments, the method may additionally comprise the step of obtaining the whole bone marrow from the animal.

The bone marrow may be enriched for hematopoietic progenitor cells by magnetic cell separation, using a lineage depletion cocktail. As will be known by the skilled person, this process is commonly used in the art for the depletion of committed leukocyte populations (i.e. mature haematopoietic cells such as T cells, B cells, NK cells, platelets etc.), thereby enabling rare cell populations such as progenitor cells to be enriched. Lineage cell depletion kits are commercially available, such as the BD IMag set from BD Biosciences, Europe.

Following the enrichment step, the hematopoietic progenitor cells remaining are incubated in the presence of IL-3. Incubation may be carried out for at least 24 hours, at least 36 hours, at least 48 hours, or at least 72 hours. In some embodiments, incubation is carried out for up to 7 days. Preferably, incubation is carried out for about 48 hours. Preferably, the IL-3 is derived from the same species from which the myeloid progenitor cells were derived. For example, in embodiments in which the myeloid progenitor cells are derived from a mouse, the IL-3 is preferably murine IL-3. It may be that the hematopoietic progenitor cells are cultured in WEHI-3B cell-conditioned medium, which provides a source of murine IL-3.

The non-human animal may be any suitable animal, such as a sheep, pig, cow, horse, goat, dog, primate, rabbit or rodent. In some embodiments, the non-human animal is a rodent (e.g. a mouse, rat, hamster, guinea pig or gerbil). The non-human animal may be a mouse.

The present invention thus provides a non-human conditionally immortalized mast cell progenitor. The progenitor cell comprises a homeobox gene, the expression of the homeobox gene being under the control of an inducer, wherein said cell expresses a heterologous high-affinity IgE receptor alpha subunit (FcεRIα). The non-human conditionally immortalized mast cell progenitor may be obtainable by the methods described herein.

Also provided is a composition comprising a population of non-human conditionally immortalized mast cell progenitors according to the invention. The composition may further comprise the inducer which controls the expression of the homeobox gene. In some embodiments the composition comprises IL-3. The composition may further comprise a suitable medium, buffer and/or salts for maintaining the cells. In some embodiments, the composition may comprise one or more cytokines (e.g. in addition to IL-3), proteins, and/or growth factors. For example, the composition may comprise one or more (or all) of: IP-10, MIP-1a, MIP-2, VEGF, IFNB-1, IL-16, IL-20, and MCP-5.

By virtue of the inducible homeobox gene, the conditionally immortalized mast cell progenitors of the invention have a high replicative rate and near-unlimited renewal potential. Advantageously, this enables on-demand differentiation to produce large numbers of mast cells within a few days. The invention thus provides a conditionally immortalized mast cell progenitor line that features remarkable self-renewal potential. The progenitor cells of the invention can be kept in culture for months without losing self-renewal potential. The progenitor cells may also be frozen (e.g. using liquid nitrogen) for storage, ensuring a stock of progenitors from which a virtually unlimited number of mast cells can be generated.

### Production of mast cells

The non-human conditionally immortalized mast cell progenitors of the invention may be cultured in the absence of the inducer and in the presence of IL-3 so as to produce differentiated mast cells. In the absence of the inducer, the progenitor cells no longer express the homeobox gene and differentiate along the myeloid lineage.

Thus, a method for preparing mast cells comprises culturing non-human conditionally immortalized mast cell progenitors which are engineered to express a heterologous high-affinity IgE receptor alpha subunit (FcεRIα), wherein the conditionally immortalized mast cell progenitors further comprise a homeobox gene, the expression of the homeobox gene being under the control of an inducer, and wherein the conditionally immortalized mast cell progenitors are cultured in the absence of the inducer and in the presence of IL-3. In some embodiments, the non-human conditionally immortalized mast cell progenitors are cultured in WEH3b cell-conditioned medium.

In some embodiments, the non-human conditionally immortalized mast cell progenitors are cultured in the absence of the inducer and in the presence of IL-3 for at least 5 days, or at least 6 days.

As described above, clonal expansion of a single non-human conditionally immortalized mast cell progenitor may be carried out to generate a monoclonal cell line from which the mast cells are generated. Thus, in any of the methods described herein which utilise mast cells, the mast cells may be monoclonal. Alternatively, the mast cells may be polyclonal.

### Cells & characterization

The present invention provides a non-human conditionally immortalized mast cell progenitor (also referred to hereinbelow as "progenitor cells"). The progenitor cell comprises a homeobox gene, the expression of the homeobox gene being under the control of an inducer, wherein said cell expresses a heterologous high-affinity IgE receptor alpha subunit (FcεRIα). The non-human conditionally immortalized mast cell progenitor may be obtainable by the methods described herein.

The invention further provides a non-human mast cell, wherein the mast cell comprises a homeobox gene, the expression of the homeobox gene being under the control of an inducer, and wherein the mast cell expresses a heterologous high-affinity IgE receptor alpha subunit (FcεRIα). The mast cell may be obtainable by the methods described herein.

Also provided is a composition comprising a population of non-human mast cells, as described herein. In some embodiments the composition comprises IL-3. The composition may further comprise a suitable medium, buffer and/or salts for maintaining the cells.

Thus, both the non-human conditionally immortalized mast cell progenitors, and the non-human mast cells derived therefrom, express FcεRIα on the cell surface. Expression of FcεRIα may be confirmed by staining the cells with an anti-FcεRIα antibody, for example using the methods described herein.

The mast cells can be distinguished from the progenitor cells from which they are derived by a number of methods. For example, the mast cells may be identified by their expression of c-kit (CD117). It has been observed that during differentiation of the progenitor cells into mast cells, by culturing the progenitor cells in the absence of the inducer, c-kit expression gradually increases over time. Thus, in a population of non-human mast cells according to the invention, at least 80%, at least 85%, at least 90%, at least 93%, at least 95%, at least 96%, at least 97%, at least 98% or at least 99% of the mast cells may be c-kit positive. In some embodiments, 100% of the mast cells in a population are c-kit positive (for example, in a monoclonal population). Mast cells which result from the differentiation of conditionally-immortalized mast cell progenitors, as described herein, may thus be identified as being c-kit and FcεRIα double positive. The mast cells may display an even distribution of both FcεRIα (e.g. human FcεRIα) and c-kit on the cell surface. c-kit expression can be detected by staining cells with anti-c-kit (e.g. anti-mouse c-kit) antibodies using well-known techniques, such as the methods described herein.

Upon differentiation of the conditionally-immortalized mast cell progenitors, the cells gradually lose Hoxb8 protein expression. This clearly distinguishes the progenitors (having high Hoxb8 protein expression) from the differentiated mast cells (low or no Hoxb8 detectable). Expression of Hoxb8 (e.g. as detected using Western Blot) may no longer be detectable by around day 2 of differentiation.

The differentiated mast cells may be further characterised by the number of FcεRIα (e.g. human FcεRIα) receptors per cell. It may be that, in the absence of IgE sensitization, the mast cells of the invention display from 5000 to 24000 receptors per cell (rpc), from 10000 to 22000 rpc, from 15000 to 20000 rpc or from 17000 to 19000 rpc, for example approximately 18000 to 18500 rpc. Incubation of the mast cells with IgE (e.g. recombinant human IgE) may increase the amount of receptors on the cells by from 4- to 6-fold, e.g. approximately 5-fold. It may be that in the presence of IgE sensitization, the mast cells of the invention display from 70000 to 120000 rpc, from 80000 to 110000 rpc, or from 85000 to 110000 rpc, e.g. about 90000 rpc.

It has been found by the present inventors that the number of receptors on the conditionally-immortalized mast cell progenitors is higher than the number of receptors on the mast cells. In the absence of IgE sensitization, the mast cell progenitors of the invention display more than 24000, more than 28000, more than 30000, or more than 32000 receptors per cell (rpc), for example from about 30000 to about 38000 or from about 32000 to about 36000 rpc (e.g. about 35000 rpc). In the presence of IgE sensitization, the mast cell progenitors of the invention display more than 90000, more than 95000 or more than 100000 rpc, for example from about 98000 to about 110000 rpc or from about 10000 to about 108000 rpc, e.g. about 106000 rpc.

Differentiated mast cells may also be identified by their morphology. Morphological analysis of the cells may be carried out by staining the cells (e.g. using toluidine blue) and/or by imaging the stained cells, for example using microscopy. Such methods will be known to the skilled person, and are described in more detail below. The mast cell phenotype is characterised by increased cellular granularity and metachromatic elements, relative to the progenitor cells from which they are derived. For example, the differentiated mast cells may be identified by the accumulation of granules, which may be observed as pink or purple dots when stained. The parent progenitor cells have no such granules.

Mast cells may further be identified by detecting the expression and/or secretion of beta-hexosaminidase, histamine, and/or mast cell proteases (e.g. mouse mast cell proteases -1, -4 and -5). These molecules are increasingly expressed (and stored in granules) upon maturation of the mast cells, and are released upon activation. The expression of mast cell proteases may be detected by detecting the corresponding mRNA (e.g. using qt-PCR, RNA sequencing or microarray), or by detecting the proteins themselves (e.g. using a western blot of cell lysates, ELISA, immunofluorescence or microscopy).

Advantageously, the non-human mast cells of the present invention may display a maximal activation of at least 50%, at least 60%, at least 70%, at least 80%, at least 85%, at least 90%, at least 92%, at least 94%, at least 95%, at least 96% or at least 97% (e.g. about 95%, or about 97%).

It has been observed that the conditionally-immortalized mast cell progenitors of the invention grow faster than previously characterised progenitor cell lines. It may be that the conditionally-immortalized mast cell progenitors of the invention have a doubling time of less than 35 hours, less than 32 hours or less than 30 hours (e.g. about 29 hours), when cultured at 37°C. Cells may be cultured with 5% CO₂ in a suitable medium, such as RPMI-1640 medium AQmedia (Sigma) supplemented with 10% FCS Sera Pro (Pan Biotech), 10% WEHI-3b supernatant, Penicillin 100U/ml, 100µg/ml Streptomycin (100x Penicillin/Streptomycin, Gibco) and 100nM 4-Hydroxytamoxifen (Sigma).

The conditionally-immortalized mast cell progenitors of the invention may be stored in culture for at least 4, at least 5, at least 6, at least 8, at least 10 or at least 12 weeks. Thus, in some embodiments fully functional mast cells can be differentiated from progenitors that have been cultured for at least 4, 5, 6, 8, 10 or 12 weeks.

The differentiated mast cells may be stored in culture (while retaining viability and functionality) for at least 5, at least 7 or at least 10 days, at 37°C. These characteristics make the mast cells of the invention particularly useful for functional assays and diagnosis.

The fact that the non-human mast cells of the invention can be derived from the same progenitor cells in a standard operating procedure makes the cells remarkably homogenous, stable and highly reproducible. Additionally, the mast cells of the invention feature an unprecedented signal-to-noise ratio upon allergen-mediated activation. While maximal activation of most previously described allergic effector cell lines lies between 40-60%, the non-human mast cells of the invention can be activated to almost 100%, indicating the dynamic range and exceptional sensitivity of the system. Importantly, these activation parameters remain constant after multiple months of progenitor cell culture, and prolonged use of the cells does not affect their viability.

### Assays

### Functional allergy assay

The mast cells of the invention find particular utility in allergy testing. In a further aspect of the invention, there is provided a method for determining whether a patient is allergic to an allergen and/or the severity of a patient's allergy to an allergen, the method comprising:
- incubating mast cells with a sample comprising patient antibodies;
- contacting the mast cells with the allergen; and
- detecting activation of the mast cells,
wherein the mast cells are non-human mast cells according to the fifth aspect of the invention. The mast cells may be obtained by the methods described herein.

Antigen or allergen mediated aggregation of FcεRIα-bound IgE on mast cells leads to their activation and immediate degranulation. Mast cells store a number of different chemical mediators, such as histamine, β-hexosaminidase, interleukins, leukotriene C4 (LTC4), proteoglycans and various enzymes, in granules. "Degranulation" is a cellular process by which, upon activation, mast cells release the contents of their granules into the surrounding environment, i.e. the surrounding tissue *in vivo,* or the cell culture supernatant in the case of an *in vitro* assay. The activation of mast cells can therefore be detected by detecting and/or quantifying chemical mediators (e.g. β-hexosaminidase) in the cell culture supernatant. Alternatively, cell surface markers that are exposed upon degranulation may be detected and/or quantified, e.g. by flow cytometry.

The mast cells and methods described herein can be used to detect allergies against any potential allergen, including air-borne allergens, food allergens (e.g. lactose, egg protein, fish, nuts, wheat and soy), drug allergens (e.g. penicillin, tetracycline, non-steroidal antiinflammatory drugs, anaesthetics), environmental allergens (e.g. pollen, birch, timothy grass, animal hair, saliva or dander, mould, latex, dust mites) and venoms (e.g. wasp and bee stings, mosquito bites).

The sample comprising patient antibodies may comprise neat or diluted patient serum. In some embodiments, the sample comprises antibodies which have been isolated from patient serum, in a suitable medium or buffer. For example, the antibodies may have been extracted from the patient serum by purification or fluid exchange.

Therefore, in any of the methods described herein, antibodies may be isolated from patient serum prior to incubating the mast cells with the patient antibodies. The antibodies may be isolated using well-known methods of antibody purification which include, but are not limited to, using protein A or protein G columns, ion exchange or metal chelate chromatography, ammonium sulfate precipitation, and Melon Gel chromatography.

In other embodiments, the patient serum is subjected to a fluid exchange process in which the fluid phase of the serum is replaced by a suitable medium, thereby obtaining a sample comprising patient antibodies. The sample containing the antibodies is then incubated with the mast cells. The fluid exchange process may be carried by spinning the serum sample through a size exclusion (e.g.100-kDa cut-off) column, into the selected medium. Preferably, the volume of serum is equal to the volume of medium so that the antibody concentration remains unchanged. This processing step ensures that antibodies remain in the medium, but that low molecular weight compounds (e.g. smaller than 100 kDa) are removed.

In any of the methods described herein, the method may further comprise:
- optionally, obtaining serum from the patient;
- isolating antibodies from the patient serum to obtain a sample comprising patient antibodies.

In any of the methods described herein, the mast cells may be incubated with the sample comprising patient antibodies for at least 8 hours, at least 10 hours, preferably at least 12 hours (e.g. overnight).

The step of contacting the mast cells with the allergen may be carried out by directly adding the allergen to the mast cell culture.

In some embodiments, the method is carried out in the absence of a wash step between the steps of incubating the mast cells with the antibodies and contacting the mast cells with the allergen. Surprisingly, a protocol in which the cells are not washed prior to allergen challenge has been found to have several advantages. Firstly, the omission of a wash step more closely mimics *in vivo* conditions in which mast cells are constantly exposed to serum. It also allows the role of allergen-specific IgG or other unknown modulatory factors in serum samples to be assessed upon allergen challenge. Furthermore, it has been unexpectedly observed that the maximal activation of the mast cells is significantly higher when cells were not washed after sensitization and before allergen challenge (Table 1). Without being bound by theory, it is thought that the IgE-allergen complex formation may occur more efficiently in the absence of a wash step, leading to enhanced cross-linking of FcεRI on the cell surface.

Detecting activation of the mast cells may comprise detecting the release of a mediator, or the expression of a surface marker, that is indicative of the presence of IgE specific for the allergen in the patient serum sample.

In some embodiments, detecting activation of the mast cells comprises detecting the expression of a surface marker. The surface marker may be a lysozyme associated membrane glycoprotein (LAMP-1, LAMP-2 or LAMP-3), CD203c, CD63 or CD107a. In some embodiments the surface marker is CD107a.

In some embodiments, detecting the expression of the surface marker comprises contacting the mast cells with an antibody specific for the surface marker, and detecting antibodies bound to the cells. For example, in embodiments therein the surface marker is CD107a, detecting the expression of the surface marker may comprising contacting the mast cells with an anti-CD107a antibody. The antibody may be added to the mast cell culture at the same time as the allergen, or after the allergen. The method may further comprise quantifying the antibodies bound to the cells, for example using flow cytometry.

In some embodiments, detecting activation of the mast cells comprises detecting the release of a mediator. The mediator may be β-hexosaminidase, a protease, histamine or a leukotriene (e.g. LTC4). In some embodiments the mediator is β-hexosaminidase. The release of β-hexosaminidase may be detected by adding a substrate of β-hexosaminidase to the culture supernatant and/or cell pellet lysates, and detecting the product of the enzyme-substrate reaction. For example, the substrate may be 4-nitrophenyl N-acetyl-β-D-glucosaminidase, which is a chromogenic substrate of β-hexosaminidase. The release of a protease may be detected using a substrate of the protease, e.g. a tryptase substrate such as acetyl-Orn-Phe-Arg-AMC, or by ELISA or immunoblot of cell supernatant.

Alternatively, mast cell activation may be detecting by detecting a pH change resulting from degranulation. Changes in pH may be detected using reagents such as pH-sensitive fluorophores or pH indicator solutions.

Detecting activation of the mast cells may comprise determining a level of activation. The level of activation may correspond to the severity of the allergy of the patient to the allergen. Thus, the level of mast cell activation can be used for clinical grading of the allergy. This, in turn, may be used to inform the type of treatment or management of the patient's allergy. The less allergen that is required to reach maximal activation of the mast cells, and/or the higher the level of maximal activation, the more allergic the patient is to the allergen. The level of activation may be determined, for example, by quantifying the amount of surface marker expressed by the mast cells, the amount of mediator released by the cells, or the extent of a pH change.

The invention thus provides a functional allergy screening assay with remarkable diagnostic potential. The methods of the invention, which are based on the passive sensitization of mast cells that are transgenic for a high-affinity IgE receptor (e.g. human FcεRIα) with IgE from patient serum, provides comprehensive information on the allergic status of the patient and overcomes many of the challenges and limitations associated with current diagnostic tools.

The functional assay of the invention, which is based on antibodies derived from serum, rather than whole blood, has the advantage that patient samples can be frozen and stored for later analysis without losing biological activity. This facilitates sample handling and allows for pro- as well as retrospective analysis of individual patient samples or entire sample cohorts.

### High-throughput multiplex assays

Conveniently, the method may be multiplexed. Thus, in some embodiments the method is for determining whether the patient is allergic to multiple allergens. In such embodiments, the method may comprise:
- separately incubating each of a plurality of mast cell populations with a sample comprising patient antibodies;
- labelling each of the plurality of mast cell populations with a different detectable label;
- after incubating the mast cell populations with the samples comprising patient antibodies, separately contacting each of the plurality of mast cell populations with a different allergen;
- pooling the plurality of mast cell populations; and
- detecting activation of the mast cells in each population,
wherein each mast cell population comprises non-human mast cells as described herein.

The step of labelling each of the plurality of mast cell populations with a different detectable label may be carried out before or after the step of incubating the plurality of mast cell populations with the samples comprising patient antibodies.

In a further embodiment, the method is for determining whether multiple patients are allergic to an allergen, the method comprising:
- separately incubating each of a plurality of samples, each sample comprising antibodies from a different patient, with one of a plurality of mast cell populations,
- labelling each of the plurality of mast cell populations with a different detectable label;
- pooling the plurality of mast cell populations;
- contacting each of the plurality of mast cell populations with the allergen; and
- simultaneously detecting activation of the mast cells in each population,
wherein each mast cell population comprises non-human mast cells as described herein.

By "each sample comprising antibodies from a different patient", it will be understood that a sample comprises multiple antibodies derived from a single patient, and that each sample is patient-specific. In this way, each patient sample is paired with a detectable label which is different to the detectable label used for each other patient. For example, the method may comprise testing whether a first patient and a second patient are allergic to an allergen. A sample comprising antibodies from the first patient may be incubated with a first population of mast cells, which are labelled with a first detectable label. A sample comprising antibodies from the second patient may be incubated with a second population of mast cells, which are labelled with a second detectable label that is different to the first detectable label. After the first and second mast cell populations are pooled and contacted with the allergen, activation of the first mast cell population can be distinguished from activation of the second mast cell population by virtue of the different detectable labels.

The step of labelling each of the plurality of mast cell population with a different detectable label may be carried out before or after the step of incubating the mast cell populations with the samples.

In some embodiments, the plurality of mast cell populations are pooled prior to contacting the mast cells with the allergen. Alternatively, the plurality of mast cell populations may be pooled after contacting the mast cells with the allergen, and before detecting activation.

In the methods described about, the mast cell populations may be labelled with any suitable detectable label, such as fluorescent labels (including fluorescently-labelled antibodies), radioactive labels, or nucleic acid (e.g. oligonucleotide) labelling.

The use of a cellular labelling or "barcoding" strategy allows simultaneous testing of multiple allergens or multiple patient sera in a high-throughput manner, resulting in a rapid and standardized diagnostic procedure. A high-throughput approach may be useful in clinical trials, for example for assessing the efficacy of drug candidates which modify serum IgE levels.

### Monitoring anti-allergy therapy

In a further aspect, the invention provides a method for monitoring the effectiveness of a therapy that is being used, that may be used in the future, or that has previously been used to treat a patient who is allergic to an allergen, the method comprising:
- incubating mast cells with a first sample comprising patient antibodies;
- contacting the mast cells with the allergen;
- determining a first level of activation of the mast cells; and
- comparing the determined first level with a reference level,
wherein the mast cells are non-human mast cells according to the fifth aspect of the invention.

In embodiments wherein the effectiveness of a current or past therapy is being monitored, the reference level may be a baseline level of mast cell activation which was determined using a sample comprising antibodies which were obtained from the patient prior (e.g. obtained from patient serum) prior to the initiation of therapy. The method may further comprise determining a reference level of mast cell activation.

In some embodiments, the method comprises providing a first sample comprising patient antibodies. The first sample may be provided by, optionally, obtaining a serum sample from the patient, and diluting the serum sample or isolating the antibodies from the serum sample (e.g. as described above).

In some embodiments, the therapy is allergen-specific immunotherapy (AIT). AIT is an important disease modifying approach for the treatment of allergic patients. The cells and methods of the invention make it possible to assess whether and when a patient is responsive to AIT, through longitudinal tracking of serum antibody reactivity. This helps to reduce or eliminate the need for *in vivo* allergen challenges to determine whether or not AIT has been successful.

The patient may undergo AIT for at least 6 months, at least 12 months, or at least 2, 3, 4 or 5 years. The patient may be currently undergoing AIT. The effectiveness of the AIT may be determined at regular intervals during the treatment period. For example, the method for monitoring the effectiveness of AIT may be carried out approximately every 3, 4, 6 or 12 months for a part or the whole of the duration of treatment, starting from initiation of the AIT. Thus, a serum sample may be obtained from the patient at 3, 4, 6 or 12 month intervals, following initiation of AIT, and antibodies present in the serum contacted with the mast cells. It may be that the effectiveness of the treatment is monitored more frequently for an initial period of time, and less frequently for a subsequent period of time. For example, monitoring may be carried out every 3 or 4 months during the first 12, 18 or 24 months of treatment, and every 6 or 12 months during the subsequent year(s) of treatment.

The method for monitoring the effectiveness of a therapy as described herein may be used to identify patients who respond to the therapy (e.g. AIT), and/or patients who do not respond to the therapy (e.g. AIT). For example, it may be that the patient has been undergoing therapy (e.g. AIT) for a period of time (e.g. 6 or 12 months, 18 months or 2 years) and no significant change in the first level of activation of the mast cells is observed relative to the reference level is observed. In such cases, it may be determined that the patient is non-responsive to the therapy (e.g. AIT). It may be that a reduction in the first level of activation of the mast cells relative to the reference level indicates that a patient is responsive to AIT. The reduction may be at least 5%, at least 7%, at least 10%, at least 15% or at least 20%. The classification of patients as responders or non-responders to therapy (e.g. AIT) can help clinicians to decide whether to proceed with therapy, or whether to use alternative treatments.

Thus, in some embodiments wherein the first sample comprises antibodies obtained from the patient at least 6 months, at least 12 months, at least 18 months, at least 20 months, or at least 24 months following initiation of AIT, and the first level of activation of the mast cells is not significantly reduced relative to the reference level, the patient is determined to be non-responsive to AIT. In such embodiments, it may be that the AIT is discontinued.

In some embodiments, the method is for monitoring the effectiveness of AIT in a patient who has completed the therapy. In other words, the method may be used to determine whether tolerance to an allergen persists after the completion of AIT. For example, the method may be used to determine whether the patient remains tolerant to the allergen at least 3 months, at least 6 months, at least 12 months, at least 1 year, at least 2 years, at least 3 years or at least 5 years after the completion of AIT. In such embodiments, a serum sample is taken from the patient at the appropriate time point, and activation of mast cells using antibodies present in the serum is tested using the method described above.

In some embodiments, the method comprises determining whether the patient has become tolerant to the allergen, i.e. as a result of the therapy. A patient may be determined as being tolerant to the allergen if the method results in substantially no activation of the mast cells.

In some embodiments, the method is for monitoring the effectiveness of a therapy which comprises treatment of the patient with an anti-allergy therapeutic agent. This may be useful for assessing the effectiveness of, for example, anti-lgE biologicals which are used to treat IgE-dependent conditions such as atopic asthma and certain food allergies. The anti-allergy therapeutic agent may be an antibody, a DARPin, an affimer, a monobody, an anticalin or an affibody.

In some embodiments the anti-allergy therapeutic agent modulates serum IgE levels and/or specificities. The anti-allergy therapeutic agent may be an anti-lgE agent. An "anti-lgE agent" is an agent which specifically binds to IgE, thereby reducing or inhibiting its function. In some embodiments, the anti-allergy therapeutic agent is an anti-lgE antibody. The anti-lgE agent may be a suppressing agent (e.g. an IgE suppressing antibody, such as Quilizumab) or a neutralizing agent (e.g. an IgE neutralizing antibody, such as Omalizumab or ligelizumab). In some embodiments, the anti-allergy therapeutic agent is an IgE modulating agent, such as Rituximab.

In some embodiments, the anti-allergy therapeutic agent is an agent which induces protective IgG.

In some embodiments, the method for monitoring the effectiveness of the therapy may further comprise detecting the induction of protective (i.e. antigen-specific) IgG. Detecting whether protective IgG has been induced in the patient may help to determine whether or not the therapy is working, and whether it should be continued. Detecting the induction of protective IgG may be carried out by:
- incubating mast cells with a second sample comprising patient antibodies, wherein the second sample is IgG-depleted;
- contacting the mast cells with the allergen;
- determining a second level of activation of the mast cells; and
- comparing the determined second level with the reference level and/or with the determined first level.

The second sample may be derived from the same serum sample as the first sample, or it may be derived from a different serum sample. IgG depletion may be carried out using Protein G- or Protein A- coupled beads (except for IgG3). For example, IgG depletion may be carried out Protein G Spin Columns (e.g. supplied by ThermoFisher).

In some embodiments the method is for monitoring the effectiveness of a potential therapy, i.e. one that may be used in the future. For example, the method may be used to assess the efficacy of a drug candidate for modifying serum IgE levels, for example as part of clinical trials.

The mast cell assay thus provides a suitable tool to functionally follow, and optionally quantify, the treatment response of allergic patients undergoing a therapy such as AIT. Not only does the assay support clinicians in discriminating responders from non-responders early on during treatment, it also aids in determining the timepoint of tolerance induction. Furthermore, the assay enables the induction of allergen-specific IgG (e.g. during AIT) to be examined, and the protective function of IgG to be assessed. The functional assay of the invention may reduce or eliminate the need for risky *in vivo* allergen challenges to be carried out.

### Further functional assays

In another aspect of the invention, there is provided a method for determining the potency of an allergen preparation, the method comprising:
- incubating mast cells with IgE specific for the allergen;
- contacting the mast cells with a sample of the allergen preparation;
- determining a level of activation of the mast cells; and
- optionally, comparing the determined level of activation with a reference level,
wherein the mast cells are non-human mast cells as described herein.

Such a method may be used for detecting variations in potency between different batches of the same allergen preparation. For example, the method may be used for the standardization of allergen preparations to be used in AIT. In some embodiments, the method is for preparing a standardised allergen preparation. The method may further comprise adjusting the potency of the allergen preparation, for example to bring the potency to within a target range.

In a further aspect of the invention, there is provided a method for allergenicity screening of a food additive or drug candidate, the method comprising:
- incubating mast cells with a sample comprising subject antibodies;
- contacting the mast cells with the food additive or drug candidate; and
- detecting activation of the mast cells,
wherein the mast cells are non-human mast cells according to the fifth aspect of the invention.

In some embodiments, the sample comprises a pool of antibodies obtained from the sera of a plurality of subjects, e.g. at least 10, 50, 100, 200, 500, 700 or 1000 subjects. In this way, the likelihood of a food additive or a drug candidate producing an allergic response in a population can be determined.

According to a further aspect of the invention, there is provided a method for determining the serum IgE concentration of a patient, the method comprising:
- incubating mast cells with a sample comprising IgE from the patient; and
- determining the amount of IgE bound to the surface of the mast cells;
wherein the mast cells are non-human mast cells according to the fifth aspect of the invention.

In some embodiments, the method is for determining the total IgE concentration in the sample. In some embodiments, the method is for determining the concentration of an allergen-specific IgE present in the sample.

Determining the amount of IgE bound to the surface of the mast cells may comprise:
- contacting the mast cells with an agent that specifically binds to the IgE;
- determining the amount of the agent bound to the cells.
The amount of agent bound to the cells may then be compared with a reference. This enables the concentration of the agent bound to the IgE, and thus the IgE concentration, to be determined.

In some embodiments, for example when the method is for determining total IgE concentration, the agent is an antibody, i.e. an anti-lgE antibody. The antibody may be labelled with a detectable label, e.g. a fluorescent dye. The concentration of the anti-lgE antibody, and thus the IgE concentration, may then be determined by detecting the detectable label on the cells, e.g. by determining the level of fluorescence using standard techniques in the art, such as FACS.

In some embodiments, for example when the method is for determining the concentration of an allergen-specific IgE, the agent is a labelled allergen (i.e. the cognate allergen for the IgE being detected). The allergen may be labelled with a detectable label, such as a fluorescent dye. The concentration of the labelled allergen, and thus the allergen-specific IgE concentration, may then be determined by detecting the detectable label on the cells, e.g. by determining the level of fluorescence. In such embodiments, the method may further comprise labelling the antigen (e.g. with a tag or fluorochrome).

The IgE concentration (total and/or allergen-specific) may be determined during or after the patient has been treated with an IgE-modulating agent, such as an anti-lgE antibody. In such embodiments, the determined IgE concentration may be compared with an IgE concentration in a sample taken from the patient prior to the initiation of treatment. This can be used to determine the effectiveness of the treatment.

The sample may comprise neat or diluted serum obtained from the patient. Alternatively the sample may comprise IgE which has been isolated from serum obtained from the patient, for example by purification or fluid exchange, in a suitable medium or buffer, as described above.

The reference may be a standard curve. A standard curve may be generated by incubating the mast cells with a series of known concentrations of IgE, contacting the mast cells with the agent (e.g. a fluorescently-labelled anti-lgE antibody or allergen), and determining the amount of agent bound to the mast cells (e.g. by determining the level of fluorescence). The amount of agent (or, for example, the level of fluorescence) can then be plotted against each IgE concentration in order to generate the standard curve.

The invention further provides a kit for allergy testing, the kit comprising:
- non-human mast cells according to the fifth aspect of the invention;
- a reagent for detecting activation of the mast cells.

In some embodiments, the kit further comprises one or more allergens, and/or a positive control. The positive control may be an anti-lgE antibody (e.g. a monoclonal antibody) which is specific for the or each allergen.

The reagent for detecting activation of the mast cells may be an antibody (e.g. an antibody specific for a lysozyme associated membrane glycoprotein (LAMP), such as CD107a or CD203c), a protease substrate (e.g a tryptase substrate), a substrate of beta-hexosaminidase or a reagent that is responsive to changes in pH (e.g. pH-sensitive fluorophores or pH indicator solutions).

In any of the aspects or embodiments described herein, the patient may be a mammal, preferably a human. The FcεRIα may be derived from the same species as the patient. Thus, it is preferred that the FcεRIα is human FcεRIα (huFcεRIα).

### EXAMPLES

The invention is further illustrated by the following examples.

### Example 1

### Materials and methods

### Reagents

Human chimeric NIP-specific JW8-IgE was purchased from NBS-C BioScience (Vienna, AUT). Human chimeric NIP-specific JW183-IgG was purchased from BioRad Laboratories (Cressier, CH). Isolated human Immunoglobulins IVIG Hizentra^{®} has been received from CSL Behring (King of Prussia, Pennsylvania, USA). Recombinant murine IL-3 was purchased from Peprotec (London, UK). Allergens NIP₇-BSA and NIP₂₄-BSA were purchased from LGC Biosearch Technologies. Various CAST^{®} Allergens in the following were received from Bühlmann Laboratories AG (Schönenbuch, CH): peanut extract (BAG-F13), cat recombinant Fel d 1 (BAG2-FELD1), wasp yellow jacket venom (BAG2-I3), honey bee venom (BAG2-I1), house dust mite extract (BAG-D1), common birch extract (BAG-T3) and timothy grass extract (BAG-G6). VivaSpin^{®} 2ml ultrafiltration spin columns, 100kDa MWCO PES membrane were purchased from Sartorius (Göttingen, GER). IgG depletion columns NAb Protein G Spin Columns, 0.2mL were purchased from Thermo Fisher Scientific. Pacific Blue^{™} Succinimidyl ester (NHS), Alexa Fluor 488 Succinimidyl ester (NHS) and Alexa Fluor 647 Succinimidyl ester (NHS) were all purchased from Thermo Fisher Scientific (Waltham, MA, USA). Hoxb8 progenitor cells were cultured in RPMI-1640 medium AQmedia (Sigma) complemented with 10% FCS Sera Pro (Pan Biotech), 10% WEHI-3b supernatant (self-made), Penicillin 100U/ml, 100µg/ml Streptomycin (100x Penicillin/Streptomycin, Gibco) and 100nM 4-Hydroxctamoxifen (Sigma). Sensitization and activation of the cells was performed in activation medium (BMMC medium) composed of RPMI-1640 w/ stable Glutamine, 2.0g/l NaHCO₃ (Seraglob) complemented with 10% Hyclone FCS (Fisher Scientific, NH, USA), penicillin 100U/ml, 100µg/ml streptomycin (100x penicillin/streptomycin, Merck, Darmstadt, Germany), 10mM HEPES buffer solution (stock-solution 1M, Life Technologies, CA, USA), 1mM Sodium Pyruvate (stock-solution 100mM, 100x, Gibco), 4mM L-Glutamine (stock-solution 200mM, 100x, Gibco), 1x Non Essential Amino Acids (stock-solution 100x, Gibco), 30ng/ml mouse recombinant IL-3 (Peprotec, London, UK), 50µM 2-Mercaptoethanol (stock-solution 14.3M, Merk). Tyrode's Buffer used for β-Hexosaminidase release assay was composed of 10mM Hepes, 130mM NaCl, 5mM KCI, 1.9mM CaCl₂, 2.1mM MgCl₂, 5.6mM L-Glucose, 0.1% BSA endotoxin-free dissolved in distilled H₂O. For flow cytometry, we used the following antibodies: anti-human IgE FITC (clone Ige21, Thermo Fisher Scientific, MA, USA), monoclonal mouse anti-human FcεRIα APC (clone AER-37, Thermo Fisher Scientific, MA, USA), monoclonal rat anti-mouse CD200R FITC (clone OX-110, AbD Serotec), monoclonal rat anti-mouse CD117 cKit PE (clone 2B8, Thermo Fisher Scientific, MA, USA), monoclonal rat anti-mouse CD107a (LAMP1) APC and PE (clone 1D4B), IgG2aκ, BioLegend, San Diego, California, USA, monoclonal mouse anti-human CD117 cKit PE (clone A3C6E2), IgG1κ BioLegend, San Diego, California, USA, monoclonal rat anti-mouse CD63 (LAMP-3) APC (clone NVG-2), IgG2aκ, BioLegend, San Diego, California, USA, And the appropriate isotype controls: mouse IgG1κ Isotype control FITC (clone P3.6.2.8.1, Thermo Fisher Scientific, MA, USA), mouse IgG2b Isotype control APC (clone eBMG2b, Thermo Fisher Scientific, MA, USA), rat IgG2bκ Isotype control PE (clone eB149/10H5), eBioscience, San Diego, California, USA, mouse IgG1κ Isotype control PE (MOPC-21), BioLegend, San Diego, California, USA. Flow cytometry was performed using a BD FACSCanto device (BD Bioscience, Franklin Lakes, NJ, USA) and results were evaluated with FlowJo Version 10.1 (Ashland, OR, USA) unless stated otherwise.

### Cell culturing of different allergic effector cells.

**Bone marrow derived mast cells (BMMCs):** Mice lacking the murine FcεRIα but transgenic for the human FcεRIα (sTG) and mice double transgenic for human IgE and human FcεRIα (dTG) were euthanized by CO₂ asphyxiation. Femur, tibia and humerus were removed and the isolated bone marrow cells were grown in BMMC medium made up of RPMI-1640 w/ stable Glutamine, 2.0g/l NaHCO₃ (Seraglob) complemented with 10% Hyclone FCS (Fisher Scientific, NH, USA), penicillin 100U/ml, 100µg/ml streptomycin (Merck, Darmstadt, Germany), 10mM HEPES buffer solution (stock-solution 1M, Life Technologies, CA, USA), 1mM Sodium Pyruvate (Gibco), 4mM L-Glutamine (Gibco), 1x Non Essential Amino Acids (Gibco), 30ng/ml mouse recombinant IL-3 (Peprotec, London, UK), 50µM 2-Mercaptoethanol (Merk). For the first two weeks, the medium was changed every second day and cells were cultured at a concentration of 2×10⁶ cells/mL in a T75 cell culture flask (Greiner Bio One, Kremsmünster, AUT) and kept in a humidified 37°C incubator with 5% CO₂. Afterwards, the medium was changed twice a week and the cells diluted to 1×10⁶ cells/ml.

**HMC-1:** HMC-1 cells were cultured at 37°C and 5% CO₂ in filter sterilized (0.22µm, Sartorius) Iscove's modified Dulbecco's medium (IMDM 1x) + GlutaMAX-I + 25mM HEPES (Gibco) supplemented with 10% Hyclone FCS (Fisher Scientific, NH, USA) and 1.2mM 1-Thioglycerol (Sigma). The cells were passaged every 3 days to be diluted to 3.5x10⁵ cells/ml and kept in a T25 cell culture flask (Greiner Bio One, Kremsmünster, AUT).

**RBL-2H3α:** RBL-2H3α cells were cultured at 37°C and 5% CO₂ in filter sterilized (0.22µm, Sartorius) RPMI-1640 medium w/ stable Glutamine, 2.0g/l NaHCO₃ (Seraglob) complemented with 10% Hyclone FCS (Fisher Scientific, NH, USA) and 500µM Geneticin G-418 Sulphate (Gibco). Cells were passaged every 3 days by removing the culture medium, two rinsing steps with 1x PBS, pH 7.4 (Insel Group) and detaching the cells for 5min at 37°C and 5% CO₂ with Trypsin-EDTA Solution 0.25% (Sigma). Trypsin-EDTA was quenched by addition of culture medium and the cells were diluted to 2x10⁵ cells/ml and kept in a T75 cell culture flask (Greiner Bio One, Kremsmünster, AUT).

**LUVA:** LUVA cells were cultured at 37°C and 5% CO₂ in filter sterilized (0.22µm, Sartorius) StemPro-34 SMF medium (Gibco) comlemented with StemPro-34 nutrient supplement (Gibco), penicillin 100U/ml, 100µg/ml streptomycin (Merck, Darmstadt, Germany), 2mM L-Glutamine (Gibco) and 100µg/ml Primocin (Invivo Gen, San Diego, California, USA). The cells were passaged every 2-3 days to be diluted to 5x10⁵ cells/ml and kept in a T25 cell culture flask (Greiner Bio One, Kremsmünster, AUT).

To assess the expression of hFcεRIα receptor on the above-mentioned cells, 5×10⁴ cells were washed twice with 200µl of PBS pH 7.4 at 600×g for 5min at 4°C. Subsequently, the cells were stained with the anti-human FcεRIα antibody or its according Isotype control antibody for 15min, light protected at RT. Flow cytometry was performed using a BD FACSCanto device (BD Bioscience, Franklin Lakes, NJ, USA) and results were evaluated with FlowJo Version 10.1 (Ashland, OR, USA).

To assess IgE-mediated activation activation of the above-mentioned cells, 5×10⁴ cells per well were seeded in a 96-well round-bottom plate. Washing was performed once with 200µl of PBS pH 7.4 at 600xg for 5min at 4°C and the cells were resuspended in 25µl corresponding medium containing increasing concentrations (0.01-50µg/ml) of JW8-IgE and incubated overnight at 37°C, 5% CO₂. Subsequently, 25µl of 2X antigen NIP₂₄-BSA diluted in medium and containing either the staining antibody anti-CD107a or anti-CD63 was added to the cells, yielding a total activation volume of 50µl and an antigen concentration for challenge of 100ng/ml. The cells were incubated for 25min at 37°C, 5% CO₂. The cells were washed twice with PBS pH 7.4 at 600xg for 5min at 4°C and measured by flow cytometry.

### Receptor quantification and IgE binding capacity of differentiated mast cells.

To quantitatively determine the surface density of the human FcεRIα on Hoxb8 mast cells the QIFIKIT^{®}(BIOCYTEX) (Code K0078, Dako, Denmark / Aligent, Santa Clara, California) was used according to the manufacturer's instructions. Therefore, 1×10⁵ differentiated Hoxb8 mast cells were incubated overnight with 5µg/ml JW8-IgE or no IgE before they were incubated with 1µg of the unconjugated monoclonal mouse anti-human FcεRIα antibody CRA-1 (Abnova, Taipeh, China) or an irrelevant monocolonal mouse anti-human CD32 (IV.3) antibody (Stem Cell Technologies, Vancouver, Canada) of the same Isotype respectively. Subsequently, the cells were stained with F(ab')₂ fragment of FITC-conjugated goat anti-mouse immunoglobulins provided with the Kit. Set-Up Beads and Calibration Beads from the Kit were prepared as recommended by the manufacturer. The amount of receptor per cell (rpc) was deduced from the calibration curve established with the MFI values of calibration bead sample.

To assess the IgE binding to the human FcεRI receptor by flow cytometry, differentiated Hoxb8 mast cells were incubated overnight with increasing concentrations (0.1-500µg/ml) of JW8-IgE. Subsequently, the cells were stained with the αhu-IgE.

To deduce IgE concentrations from human serum samples by flow cytometry, differentiated Hoxb8 mast cells were incubated overnight with 25 different human serum samples used undiluted and in dilutions of 1:2, 1:5, 1:10. Afterwards, cells were proceeded as described above. IgE concentrations in the serum samples were calculated from data interpolation into the standard curve established with IgE-JW8 using GraphPad, Prism 8 (GraphPad Holdings LLC, San Diego, California, USA).

### Activation of differentiated mast cells with recombinant proteins and patient sera.

For the determination of the Hoxb8 mast cell activation by flow cytometry, differentiated Hoxb8 mast cells were incubated overnight with increasing concentrations (0.01-5µg/ml) of JW8-IgE for cells sensitization. Subsequently, the cells were stimulated by direct addition of 100ng/ml of the antigen NIP₂₄-BSA and the staining antibody anti-CD107a. Only now, the cells were washed to prepare for acquisition.

Differentiated Hoxb8 mast cells were treated as described above for overnight IgE-JW8 sensitization and then stimulated with 100ng/ml of the antigen NIP₇-BSA diluted in Tyrode's buffer (10mM Hepes, 130mM NaCl, 5mM KCI, 1.9mM CaCl₂, 2.1mM MgCl₂, 5.6mM L-Glucose, 0.1% BSA endotoxin-free dissolved in distilled H₂O) was added. The cells were incubated 60min at 37°C, 5% CO₂. Subsequently, the cells were centrifuged at 600xg for 5min at 4°C. 50µl of the supernatants were transferred to a 96-well flat-bottom plate, while the remaining supernatants were removed from the cells. Then the cells were lysed by addition of 100µl 0.5% Triton-X made up in Tyrode's buffer and resuspended thoroughly. Again, 50µl of the pellet lysate was transferred to the 96-well flat-bottom plate. 50µl of the substrate pNAG solution (4mM 4-nitrophenyl N-acetyl-B-D-glucosaminidase in substrate buffer containing 201mM Na₂HPO₄ and 438mM citric acid in ddH₂O, pH 4.5, Sigma) were added to the supernatants and pellet lysates and incubated for 60min at 37°C (no CO₂). The enzyme-substrate reaction was stopped by addition of 100µl 0.2M glycine pH 10.7 to each well and the absorbance at 405nm wavelength was measured on a standard ELISA plate reader SpectraMax M5 (Molecular Devices LCC, San Jose, California, USA). The percentage of the net release of β-hexosaminidase from the RBL-2H3α cells upon challenge was calculated by dividing the amount of released by the total amount of β-hexosaminidase (released plus leftover in the cells after challenge).

Serum samples from patients with defined allergies against peanut, cat, wasp, honey bee, house dust mite, birch and timothy grass were prepared for Hoxb8 mast cell sensitization employing VivaSpin^{®} 2ml ultrafiltration spin columns (MWCO 100kDa). The columns were used according to the manufacturer's instructions to perform a buffer exchange of the human serum samples with BMMC medium. To determine the activation of Hoxb8 mast cell passively sensitized with human patient serum by flow cytometry, 5×10⁴ differentiated Hoxb8 mast cells per condition were resuspended in processed serum samples and incubated overnight for IgE-sensitization of the cells. Subsequently, the cells were either washed prior to the addition of the cognate antigen and the staining antibody anti-CD107a, or the wash step was omitted (as indicated). The allergens were titrated and the cells challenged with 1-50ng/ml allergen for peanut, 0.1-100ng/ml for cat, 1.1-100ng/ml for wasp, 1-100ng/ml for honey bee, common birch and timothy grass, and 3.7-1000ng/ml for house dust mite. The cells were incubated for activation, then washed and acquired.

To assess the effect of allergen-specific IgG possibly present in allergic patients after AIT, an artificial serum has been assembled by mixing 240ng/ml JW8-IgE with 10mg/ml IVIG human immunoglobulins, either in presence or absence of 50µg/ml JW183-IgG. These artificially assembled serum samples were added to differentiated Hoxb8 mast cells and incubated overnight. Subsequently, the cognate antigen NIP₇-BSA and the staining antibody anti-CD107a were added to the cells for stimulation. The antigen was added in increasing concentrations of 0.07ng/ml-333.33µg/ml to assess dose-dependent activation. Following the stimulation, the cells were washed and cell activation was assessed by flow cytometry using a CytoFLEX S 4L 13C (B2-R3-V4-Y4) plus 96 DW plate loader, Beckman Coulter Life Sciences (Brea, California, USA). Results were evaluated with FlowJo Version 10.1 (Ashland, OR, USA).

Serum or plasma samples from SCIT patients at time points 0 (pre-treatment) as well as 3, 6, 9, 12, 24 and 36 months of therapy (post-treatment) were prepared for Hoxb8 mast cell sensitization with VivaSpin^{®} 2ml ultrafiltration spin columns (MWCO 100kDa) as described above. To determine the effect of SCIT on the activation of Hoxb8 mast cell passively sensitized with human patient serum, differentiated Hoxb8 mast cells were incubated overnight with the according processed serum samples. Subsequently, the cognate antigen timothy grass was added to the cells for stimulation in increasing concentrations in the range of 5-100ng/ml together with the staining antibody anti-CD107a. Only after stimulation, the cells were washed and acquired.

To investigate the effect of IgG in the serum of SCIT patients, IgG was removed from serum samples of patients post 12 months of therapy using NAb Protein G Spin Columns (Thermo Fisher Scientific) according to the manufacturer's instructions. Two runs of IgG-depletion were performed on 400µl of serum before proceeding with the buffer exchange step using the VivaSpin^{®} 2ml ultrafiltration spin columns as described above. Sensitization and activation of differentiated Hoxb8 mast cells was performed as before.

### Fluorescent cell barcoding of differentiated mast cells.

In order to design a setup to allow a high-throughput format for the Hoxb8 mast cell activation, differentiated Hoxb8 mast cells labeled with 36 (4x3x3) unique fluorescent barcodes. Therefore, all possible concentration combinations of the fluorescent dyes Pacific Blue succinimidyl ester (40, 6, 0.5, 0µg/ml), Alexa Fluor 488 succinimidyl ester (40, 2, 0µg/ml) and Alexa Fluor 647 succinimidyl ester (8, 0.5, 0µg/ml) were prepared in PBS pH 7.4 and added to the cells. For covalent amine-coupling, the cells were incubated for 25min at RT according to the manufacturer's instructions. Subsequently, the cells were washed before being pooled in a 5ml FACS tube for flow cytometry measurements were performed using a BD FACS LSR II SORP device (BD Bioscience, Franklin Lakes, NJ, USA). Single cell populations were deconvolved using FlowJo Version 10.1 (Ashland, OR, USA).

To assess activation of Hoxb8 mast cells in the high-throughput format, differentiated Hoxb8 mast cells per well were incubated overnight with increasing concentrations (0.01-2µg/ml) of JW8-IgE for sensitization. Then, the cells were washed before labeling with 9 (3x3) unique fluorescent barcodes was performed as described above using the fluorescent dyes Alexa Fluor 488 succinimidyl ester (40, 2, 0µg/ml) and Alexa Fluor 647 succinimidyl ester (0.5, 0µg/ml). Following the covalent amine-coupling the cells were washed prior to the addition of the cognate antigen NIP₇-BSA at 100ng/ml and the staining antibody anti-CD107a. After activation of the single cell conditions, the cells were washed, pooled a 5ml FACS tube and flow cytometry was performed using a BD FACS LSR II SORP device (BD Bioscience, Franklin Lakes, NJ, USA). The barcoded cell populations were deconvolved and activation of each population was determined with FlowJo Version 10.1 (Ashland, OR, USA).

To demonstrate the high-throughput format for the activation of Hoxb8 mast cells after passive sensitization with serum samples from human allergic patients, differentiated Hoxb8 mast cells were incubated overnight with VivaSpin^{®} 2ml ultrafiltration spin columns (MWCO 100kDa) processed serum samples from 8 defined timothy grass allergic patients. Then, washing was performed prior to the labeling the Hoxb8 mast cells with 36 (4x3x3) unique fluorescent barcodes with the fluorescent dyes Pacific Blue succinimidyl ester (40, 6, 0.5, 0µg/ml), Alexa Fluor 488 succinimidyl ester (40, 2, 0µg/ml) and Alexa Fluor 647 succinimidyl ester (8, 0.5, 0µg/ml). Subsequently, the cells were washed and the 9 conditions with the same Pacific Blue barcode were pooled into one 5ml FACS tube. For activation, one concentration of the cognate antigen timothy grass (0, 10, 50, 100ng/ml) and the staining antibody anti-CD107a were separately added to one of the 4 FACS tubes. After activation, washing was performed for the 4 FACS tubes and once more after pooling the cells of all 4 tubes into a single one. Flow cytometry was performed using a BD FACS LSR II SORP (upgrade) device (BD Bioscience, Franklin Lakes, NJ, USA) and results were evaluated with FlowJo Version 10.1 (Ashland, OR, USA). The barcoded cell populations were deconvolved and activation of each population was determined with FlowJo Version 10.1 (Ashland, OR, USA).

### Generation and differentiation of conditional Hoxb8-immortalized progenitors

Subcloning of the mouse Hoxb8 coding sequence into the pF-5xUAS-SV40-puro-Gev16 lentiviral vector system and generation of viral particles has been described elsewhere^{20,21}. In short, lentiviral particles carrying a 4-hydroxytamoxifen (4-OHT) inducible Hoxb8 expression system were produced in HEK 293T cells by transient transfection using X-tremeGENE HP transfection reagent (Roche Diagnostics, Rotkreuz, CH). A total of 15 µg DNA was transfected per 10-cm tissue culture dish, using a ratio of pMD2.VSV-G (envelope proteins):pCMVδR8.2 (packaging elements):pF-5xUAS-Hoxb8(mm)-Sv40puroGev16 of 2:5:3 with 30 µl of transfection reagent. Medium was replaced the next day and virus-containing supernatant collected 24 h and 48 h later. Supernatants were pooled and passed through a 0.2 µm filter and used fresh for infection. Hematopoietic progenitor cells were enriched from bone marrow of B6.Cg-*Fcer1a^{tm1Knt}* Tg(FCER1A)1Bhk/J mice by magnetic cell separation using a lineage depletion cocktail (BD IMag^{™}, BD Biosciences Europe) following the manufacturer's instructions. 5x10⁵ lineage-marker depleted cells were incubated for 48 h in complete RPMI medium (RPMI^{c}= RPMI-1640 AQmedia^{™}, 10% FCS, 1% Penicillin/Streptomycin, 50 µM 2-mercaptoethanol) in the presence of 300-400 pg/ml IL-3 (added as WEHI-3B cell conditioned medium as a source for murine IL-3). Cells were spin infected with ^{cond}Hoxb8 lentiviral particles (1 mL virus containing supernatant, supplemented with 8 µg/ml polybrene) at 30°C for 90 min. Cells were transferred to RPMI^{c}/IL-3 medium and Hoxb8 expression induced by addition of 0.1 µM) 4-OHT. Puromycin selection (1 µg/ml) was started 4 days after infection and maintained until outgrowth of surviving cells. Obtained cell lines were cultured in RPMI^{c}/IL-3/4-OHT and selected based on combination of best growth, viability and functional performance in the functional assay. The best performing line was furthermore used for testing of subclones by limited single cell dilution and clonal expansion in 96-well plates using 50% pre-conditioned growth medium. To differentiate progenitors into mature allergic effector cells, cells were washed twice in PBS and reseeded at 7.5x10⁴ cells/ml in RPMI^{c}/IL-3 medium for 5-6 days.

### Flow cytometric and morphological characterization of cells.

To characterize the Hoxb8 mast cell progenitor line at various stages of their differentiation, the cells were stained at day 0, 2, 4 or 6 after differentiation start with anti-mouse cKit CD117 and anti-human FcεRIα antibodies. To visualize the expression of the surface markers and IgE binding to huFcεRIα in a spatial context by multispectral imaging flow cytometry, 1x10⁵ differentiated Hoxb8 mast cells were incubated with anti-mouse cKit CD117 and anti-human FcεRIα antibodies, as well as the according isotypes. Hoxb8 mast cells after overnight incubation with 0, 0.5, 5µg/ml JW8-IgE were stained with anti-human IgE and anti-human FcεRIα antibodies. Expression and distribution of the surface marker anti-mouse cKit and anti-human FcεRIα and the binding of IgE-JW8 to the receptor was assessed using an Amnis^{®} ImageStream^{®}X MKII and the corresponding IDEAS^{®} software (Luminex corporation, Austin, TX, USA).

To visualize the progressive increase in cellular granularity of the Hoxb8 mast cell progenitor line trough differentiation, 5×10⁴ cells were immobilized by use of a Cellspin^{®}I centrifuge (Tharmac, Wiesbaden, Germany) on microscopy slides (Thermo Fisher Scientific, Waltham, Massachusetts, USA) at day 0, 2, 4 or 6 after differentiation start. Cells were stained with 1% Toluidine Blue in methanol solution (Insel Apotheke), dehydrated, cleared and mounted with Cyto Seal XYL^{™} (Thermo Fisher Scientific, Waltham, Massachusetts, USA). Images were acquired in brightfield mode using the HCX PL APO objective for 63x magnification at the LEICA DMI4000 microscope and the corresponding LAS V4.2 software.

### Human samples and animals

Human serum samples of allergic donors were received from the Center of Laboratory Medicine at the University Hospital Bern with approval from the local ethics committee (KEK 2017-01590). Human serum samples of allergic donors that underwent allergen-specific subcutaneous immunotherapy were received from an study approved from the Regional Committee on Biomedical Research Ethics (M2009-0121) at Aarhus University Hospital. Informed consent was obtained in accordance with the Helsinki Declaration. Mice transgenic for human FcεRIα and with the murine FcεRIα knocked out were obtained from Prof. Jean-Pierre Kinet. Double transgenic mice expressing human IgE and human FcεRIα were licensed from GenOway S.A. All animal experimentation was approved from the local animal committee (BE66/18).

### Statistics

Statistical analysis and calculation of linear as well as non-linear fitting models as indicated in the figure legends were carried out in Prism 8.0 software (GraphPad Software, La Jolla, Calif). Wherever suitable individual datapoints are shown. For all other graphs, data are displayed as mean ± SEM.

### Results

### Generation and differentiation of Hoxb8 immortalized progenitor cells

To generate a virtually unlimited source of allergic effector cells to be used in a functional diagnostic test for human IgE-dependent type-1 hypersensitivity reaction, we sought to conditionally immortalize mast cell progenitors from mice that are transgenic for the human high-affinity IgE receptor (huFcεRIα^{tg}). For this purpose, we isolated whole bone marrow from the femurs of huFcεRIα^{tg} mice, performed a linage depletion (i.e. removal of committed leukocyte populations) and cultured the remaining myeloid progenitor cells for two days in differentiation medium containing murine IL-3 (**Fig 1****, *A***)*.* In a next step, we spin-infected these cells with a previously described 4-hydroxytamoxifen (4-OHT) inducible homeobox B8 (Hoxb8) expression system including a puromycin resistance gene. Antibiotic pressure in the presence of 4-OHT and murine IL-3 containing differentiation medium resulted in the selection of immortalized progenitor cells with extensive growth and self-renewal potential.

It has previously been demonstrated that shut-down of exogenous Hoxb8 expression upon withdrawal of 4-OHT in such stably transduced progenitor cultures readily induces cell differentiation along the myeloid lineage. Depending on the cytokines present, generation of neutrophils, macrophages and basophils has been described. Thus, we first assessed whether our progenitor cells might differentiate into mature allergic effector cells (i.e. basophils or mast cells) in the presence of IL-3. Importantly, the generation of mast cells using conditional Hoxb8 has not yet been reported. Indeed, we observed differentiation into allergic effector cells upon 4-OHT removal from the culture medium (**Fig. 1****, B**). We decided to further proceed with the cells showing the most uniform expression of c-kit. With the goal to ultimately obtain a robust mast cell progenitor line, we characterized these cells at various stages of differentiation (i.e. day 0-6). Flow cytometric analysis demonstrates that c-kit expression gradually increased over time and that by day 6 more than 90% of the cells were c-kit and huFcεRIα double-positive (**Fig 1****, *B***). To further visualize these cell surface markers in a spatial context, we additionally performed image stream flow cytometry. These measurements revealed an even distribution of both huFcεRIα and c-kit on the cell surface and confirmed up-regulation of c-kit upon differentiation. Toluidine blue staining of the cells at various differentiation stages additionally indicates a progressive increase of cellular granularity and a clear mast cell phenotype with metachromatic elements (**Fig 1****, *C***). In addition, we performed limited dilution of the selected polyclonal progenitor cells and subsequent clonal expansion that resulted in the generation of a monoclonal Hoxb8 mast cell line (i.e. NT-1). NT-1 showed remarkably similar characteristics as the parental polyclonal line in terms of c-kit/huFcεRIα expression and IgE-binding. We ultimately decided to continue using the polyclonal line for subsequent experiments. Together, this data provides strong evidence that our selection strategy resulted in the identification of an immortalized huFcεRIα transgenic mast cell progenitor line, from which differentiated Hoxb8 mast cells can be derived in as little as 5 days upon removal of 4-OHT from the cell culture in the presence of murine IL-3 containing differentiation medium.

### Cellular and functional characterization of differentiated Hoxb8 mast cells

To further characterize differentiated Hoxb8 mast cells, we quantified the absolute amount of huFcεRIα receptors per cell (rpc) and compared it to levels measured on either bone marrow derived mast cells from single transgenic huFcεRIα mice (i.e. BMMCα-sTG), double transgenic huIgE/huFcεRIα mice (i.e. BMMCα-dTG), rat basophilic leukemia cells that were stably transfected with huFcεRIα (i.e. RBL-2H3α) and the human mast cell lines HMC-1 and LUVA (**Fig 2**, ***A*** and see **Table 1**). In the absence of IgE sensitization, Hoxb8 mast cells display an average of 18'415 rpc, while BMMCα-sTG express almost twice as many receptors with 35'160 rpc. BMMCα-dTG showed an intermediate level with 25'007 rpc and RBL-2H3α a low amount with 1'084 rpc. Surface expression of huFcεRIα was undetectable on both HMC-1 and LUVA. Overnight incubation in the presence of 5 µg/ml recombinant human IgE (hulgE) increases the amount of huFcεRIα about 5-fold on every cell line that has been analyzed (**Fig 2****, *A*** and see **Table 1**). This finding is in line with previous reports demonstrating that IgE stabilizes its receptor on the mast cell surface²³. Image stream flow cytometry analysis further reveals a homogenous distribution and co-localization of huFcεRIα with hulgE. No clustering or aggregation on the Hoxb8 mast cell surface has been observed. Titration experiments with recombinant human IgE-JW8 on Hoxb8 mast cells demonstrates that IgE can be detected at concentrations in a linear range from >1 µg/ml to <100 µg/ml by flow cytometry. Thus, this assay is able to detect IgE binding with a dynamic range of more than 2 logs (**Fig 2****, *B***). Next, we assessed whether it is possible to use the Hoxb8 mast cell system to determine total IgE concentrations in human serum samples. Using standard curves, we quantified IgE levels in sera from 25 individuals on Hoxb8 mast cells and compared those results to total serum IgE values measured by the current gold-standard singleplex immunoassay (i.e. ImmunoCAP^{™}, Phadia). Remarkably, the results demonstrate that the Hoxb8 mast cell system is accurate and shows a high degree of correlation (r = 0.972, *p*<0.0001) with the measurements by singleplex immunoassay (**Fig 2****, *C***).

**Table 1: Characterisation of different allergic effector cells**

| **Characteristics** | **Hoxb8 mast cells** | **BMMCα-sTG** | **BMMCα-dTG** | **HMC-1** | **RBL-2H3α** | **LUVA** |
|---|---|---|---|---|---|---|
| **FcεRIα expression** | Yes | Yes | Yes | No | Yes | No |
| **Number of FcεRIα per cell (absence IgE)** | 18'415 | 35'160 | 25'007 | - | 1'084 | - |
| **Number of FcεRIα per cell (presence IgE)** | 90'831 | 200'374 | 188'475 | - | 5'519 | - |
| **Maximal Activation no wash (Activation Marker)** | -95% (CD107a) | ∼60.8% (CD107a) | ∼67.5% (CD107a) | - | - (CD63/CD107a) | - |
| **Maximal Activation with wash (Activation Marker)** | -60% (CD107a) | ∼41.5% (CD107a) | ∼41.5% (CD107a) | - | - (CD63/CD107a) | - |

It is well established that antigen or allergen mediated aggregation of FcεRIα-bound IgE on mast cells leads to their activation and immediate degranulation. However, to be functionally active huFcεRIα has to pair with murine γ-chains containing an intracellular signaling domain (i.e. formation of the trimeric αγ₂ receptor) and ideally associate with the murine β-chain also containing an intracellular signaling domain and serving as a signal amplifier (i.e. formation of the tetrameric αβγ₂ receptor). To assess the expression of α-, β- and γ-chains in Hoxb8 mast cells we performed western blot analysis. All three chains are detectable, strongly suggesting that the heterotetrameric αβγ₂ receptor form is expressed on the surface of Hoxb8 mast cells. Activation and degranulation of allergic effector cells can be assessed by various means. On the one hand, researchers quantify specific enzymes or mediators (e.g. β-hexosaminidase) that get released upon degranulation in the cell culture supernatants. Alternatively, cell surface markers that get exposed upon degranulation, such as the lysosome-associated membrane protein-1 (LAMP-1 or CD107a), may be quantified by flow cytometry. To assess whether huFcεRIα in differentiated Hoxb8 mast cells is functionally active, we sensitized cells with increasing concentrations of antigen-specific IgE-JW8 and challenged them with a fixed concentration of the cognate antigen (NIP₂₄-BSA). Using cell surface CD107a positivity as a read-out (**Fig 2****, *D***) our results indicate dose-dependent activation of Hoxb8 mast cells, which is highly reproducible in its maximal activation and which overall shows a remarkable signal-to-noise ratio (**Fig 2****, *E***). While maximal activation is reached at -95% - a value that has previously neither been observed with mast cell lines nor with bone marrow derived mast cells (**Table 1**) - background activation remains low at <1%. We also tested Hoxb8 mast cells derived from a monoclonal progenitor line (i.e. NT-1), which behaves almost identically to cells derived from its parental polyclonal line. Besides the exceptionally high maximal activation values, Hoxb8 mast cells show other favorable characteristics. The total yield of differentiated cells after 5 days is 5.8-times the number of seeded progenitor cells (**Fig. 2****, *F***), which is in a similar range as previously described for human blood-derived mast cell cultures. However, with a calculated doubling time of 28.8 hours, the HoxB8 progenitor cultures grow clearly faster than other previously described mast cell or basophil lines including BMMCα-sTG, BMMCα-dTG, RBL-2H3α, HMC-1 or LUVA (**Fig 2****, G**). Importantly, HoxB8 mast cells are still functional after 5 weeks of progenitor cell culturing without losing activity (**Fig 2****, *H***). Since previous studies involving in-vitro models of mast cell activation preferentially used quantification of β-hexosaminidase in the supernatants rather than flow cytometric analysis of activation markers, we also assessed how these two parameters relate to each other for Hoxb8 mast cells. By performing both measurements we find that there is a close correlation of released β-hexosaminidase and exposed CD107a surface marker (**Fig 2****, *I***).

The differentiated Hoxb8 mast cells were found to have exceptional survival and maintenance of reactivity in the assay of at least 7 days in differentiation medium at room temperature without O₂ or CO₂ supplementation in closed tubes. This finding underscores the robustness of the cells.

### Probing for IgE-mediated reactions using Hoxb8 mast cells

We have shown that IgE from human serum readily binds to huFcεRIα on differentiated Hoxb8 mast cells and demonstrated that these cells immediately degranulate upon IgE cross-linking by antigenic stimuli. Next, we wanted to assess whether passive sensitization of Hoxb8 mast cells with pre-defined sera from allergic patients could be used to test allergen-specific activation. To get a representative picture for different allergen sources, we incubated Hoxb8 mast cells with sera from either peanut, cat, wasp, honey bee, house dust mite, birch or timothy grass allergic individuals and additionally determined total and allergen-specific IgE in these samples by singleplex immunoassay (**Table 2**). We additionally made sure that sera from different RAST classes are represented in the tested samples. For all allergens used, we observed dose-dependent activation of Hoxb8 mast cells (**Fig 3****, *A-G***), suggesting that this experimental setup is suitable to screen patients for unknown allergies. Interestingly, activation did not correlate with the amount of either allergen-specific or total IgE in the serum, nor with the ratio thereof, indicating that additional parameters such as the presence of protective allergen-specific IgG influence the outcome in this functional assay. These data strongly suggest that the Hoxb8 mast cell assay can be used to identify IgE-mediated allergies to virtually any allergen as well as to determine the severity of the allergic response based on maximal activation at a given allergen concentration.

**Table 2: Samples for allergy screening**

| **Sample** | **Total IgE** | **Specific IgE** | **Ratio %** | **RAST class** | **Allergen** |
|---|---|---|---|---|---|
| 1 | 91 | 11.5 | 12.637 | 3 | rAra h2 (Peanut) |
| 2 | 259 | 37.8 | 14.595 | 4 | rAra h2 (Peanut) |
| 3 | 565 | 61.7 | 10.920 | 5 | rAra h2 (Peanut) |
| 4 | 851 | 100 | 11.751 | 6 | rFel d1 (Cat) |
| 5 | 409 | 33.6 | 8.215 | 4 | rFel d1 (Cat) |
| 6 | 385 | 58.7 | 15.247 | 5 | rFel d1 (Cat) |
| 7 | 200 | 34.34 | 17.170 | 4 | rVes v1 and rVes v5 (Wasp) |
| 8 | 151 | 51.92 | 34.384 | 4 | rVes v1 and rVes v5 (Wasp) |
| 9 | 62 | 16.21 | 26.145 | 3 | rVes v1 and rVes v5 (Wasp) |
| 10 | 83 | 11.3 | 13.614 | 3 | rApi m1 (Honey Bee) |
| 11 | 258 | 48.4 | 18.760 | 4 | rApi m1 (Honey Bee) |
| 12 | 434 | 59.9 | 13.802 | 5 | rApi m1 (Honey Bee) |
| 13 | 132 | 11.5 | 8.712 | 3 | nDer p1 & nDer p2 (house dust mite) |
| 14 | 708 | 99.1 | 13.997 | 5 | nDer p1 & nDer p2 (house dust mite) |
| 15 | 1339 | 155.3 | 11.598 | 6 | nDer p1 & nDer p2 (house dust mite) |
| 16 | 405 | 100 | 24.691 | 6 | rBet v1 (Birch) |
| 17 | 118 | 26.3 | 22.288 | 4 | rBet v1 (Birch) |
| 18 | 145 | 6.34 | 4.372 | 3 | rBet v1 (Birch) |
| 19 | 146 | 16.8 | 11.507 | 3 | rPhl p1 & rPhl p5b (Timothy Grass) |
| 20 | 764 | 100 | 13.089 | 6 | rPhl p1 & rPhl p5b (Timothy Grass) |
| 21 | 494 | 100 | 20.243 | 6 | rPhl p1 & rPhl p5b (Timothy Grass) |
| 22 | 198 | 34.2 | 17.273 | 4 | rPhl p1 & rPhl p5b (Timothy Grass) |
| 23 | 883 | 100 | 11.325 | 6 | rPhl p1 & rPhl p5b (Timothy Grass) |
| 24 | 845 | 100 | 11.834 | 6 | rPhl p1 & rPhl p5b (Timothy Grass) |

### Monitoring AIT with differentiated Hoxb8 mast cells

It is well understood that the induction of protective IgG represents one of the mechanisms underlying the establishment of tolerance upon allergen-specific immunotherapy (AIT). Nevertheless, there is no standardized and validated functional assay available to test the implications of allergen-specific IgG induction and to monitor treatment success of AIT. Here, we addressed the question of whether passive sensitization of Hoxb8 mast cells with patient sera could be useful for the monitoring of allergic patients undergoing AIT and whether this approach might help to predict treatment outcome. As a proof-of-concept, we sensitized Hoxb8 mast cells with a fixed concentration of recombinant NIP-specific human IgE-JW8 in the presence or absence of a 200-fold excess of NIP-specific IgG. Upon activation of the cells with different concentrations of NIP₇-BSA antigen, we observed a shift of the activation curve towards higher antigen concentrations for the NIP-specific IgG containing sample. Additionally, a decrease in maximal activation became apparent if NIP-specific IgG was present (**Fig 4****, *A***). Next, we tested serum samples of timothy grass allergic patients that had undergone AIT over the course of at least 36 months (**Table 3**). This longitudinal observation revealed that patient sera showing reactivity on Hoxb8 mast cells to timothy grass at baseline (i.e. before treatment initiation) became unresponsive after 12 months of treatment or later timepoints (**Fig 4****,** *B*). On the other hand, serum from a placebo treated control patient showed no obvious change in reactivity over the timeframe of 36 months. To assess whether unresponsiveness of patient sera post-SCIT were due to the presence of protective allergen-specific IgG, we depleted IgG from 12 month post-SCIT sera and compared them to untreated 12 month post-SCIT sera. While the untreated SCIT sera induced as expected no activation, the IgG depleted sera became highly reactive and dose-dependently activated the Hoxb8 mast cells upon timothy grass allergen challenge (**Fig 4****, *C***). These data strongly indicate that the functional Hoxb8 mast cell assay is helpful in longitudinal monitoring of patients undergoing AIT treatment and for the clinical interpretation of therapy outcome. Further, our findings re-emphasize the importance of allergen-specific IgG induction during SCIT that has previously been reported.

**Table 3: Samples for SCIT screening.**

| **Sample** | **Specific IgE (pre-treatment)** | **Specific IgE (post treatment)** | **RAST class** | **Specific IgG4 (pre-treatment)** | **Specific IgG4 (post-treatment)** | **Allergen** |
|---|---|---|---|---|---|---|
| 1 | 32.8 | 48.5 | 4 | 0.01 | 0.7 | rPhl; all major and minor (Timothy Grass) |
| 2 | 42.2 | 35.5 | 4 | 0.1 | 6.8 | rPhl; all major and minor (Timothy Grass) |
| 3 | 25.3 | 201.2 | 4 | 0.1 | 8.1 | rPhl; all major and minor (Timothy Grass) |
| Ctrl | 34.6 | 41.2 | 4 | 0.01 | 0.01 | rPhl; all major and minor (Timothy Grass) |

### High-throughput allergy screening with differentiated Hoxb8 mast cells

In a next step we investigated whether an allergy screening assay based on Hoxb8 mast cells activation could be adapted onto a high-throughput format. For this purpose, we labeled sensitized cells with varying concentrations of fluorescent dyes (i.e. Pacific Blue, Alexa Fluor 488, Alexa Fluor 647) using covalent amine-coupling protocols (**Fig 5**, ***A(i)***)***.*** However, it will be appreciated that in alternative embodiments the mast cells could be labelled prior to sensitization (**Fig. 5****, *A(ii)***)*.* Each batch of cells receives a unique cellular barcode based on a particular fluorescent label. Flow cytometric analysis of the Hoxb8 mast cells reveals a nice separation of the differentially labeled cell populations (**Fig 5****, *B***). As a gating strategy, we chose to first separate SSC^{high}/FSC^{high} viable cells according to their pacific blue fluorescence intensity and subsequently to plot Alexa Fluor 488 against Alexa Fluor 647 signals. In a proof-of-concept experiment, we used different concentrations of recombinant human NIP-specific human IgE-JW8 to sensitize individually labeled cell populations before pooling all of them in a single tube to which we subsequently added a fixed concentration of NIP₇-BSA antigen for activation. Flow cytometric acquisition and analysis allowed us to retrieve the individual cell populations and monitor their activation status (**Fig 5****, *C***). The percentage of activation as determined by CD107a cell surface positivity of each identified cell population nicely correlated with the amount of sensitization (**Fig 5****, *C-D***). In a next step, we used eight pre-defined sera (**Table 4**) from timothy grass allergic patients to sensitize individually labeled cell populations before pooling those in four tubes. To each of the four tubes a certain concentration of timothy grass extract (i.e. 0, 10, 50, 100ng/ml) was added for activation. All cells were pooled for acquisition and subsequently deconvoluted to identify individual cell populations based on their fluorescence barcode (**Fig 5****, *E***). Each serum sample except for the no serum control shows allergen dose-dependent cell activation (**Fig 5****, *F***), strongly supporting that the fluorescent cell barcoding based high-throughput approach is suitable to screen multiple sera in one experimental run.

**Table 4: High-throughput screening samples**

| **Sample** | **Total IgE** | **Specific IgE** | **Ratio %** | **RAST class** | **Allergen** |
|---|---|---|---|---|---|
| A | 146 | 16.8 | 11.507 | 3 | rPhl p1 & rPhl p5b (Timothy Grass) |
| B | 198 | 34.2 | 17.273 | 4 | rPhl p1 & rPhl p5b (Timothy Grass) |
| C | 385 | 72.1 | 18.727 | 5 | rPhl p1 & rPhl p5b (Timothy Grass) |
| D | 477 | 66.6 | 13.962 | 5 | rPhl p1 & rPhl p5b (Timothy Grass) |
| E | 764 | 100 | 13.089 | 6 | rPhl p1 & rPhl p5b (Timothy Grass) |
| F | 883 | 100 | 11.325 | 6 | rPhl p1 & rPhl p5b (Timothy Grass) |
| G | 494 | 100 | 20.243 | 6 | rPhl p1 & rPhl p5b (Timothy Grass) |
| H | 845 | 100 | 11.834 | 6 | rPhl p1 & rPhl p5b (Timothy Grass) |

Next, we investigated whether an individual patient serum could be screened against multiple allergens in such a high-throughput approach. To do so, we incubated barcoded cells with processed sera from two polysensitized patients (Table 5) and separately stimulated them with various allergens. After activation, cells from an individual patient serum were pooled for acquisition and subsequently deconvoluted to identify individual cell populations based on their fluorescence barcode. While we detect reactivity against grass-mix as well as cat and birch allergens in serum of patient 1 (**Fig. 6****, A** and **C**), the serum of patient 2 reacts against house dust mite and cat allergens (**Fig. 6****, B** and **D**). These data clearly indicate that the established high-throughput format is a suitable and rapid set-up to functionally screen and identify polysensitized patients.

**Table 5: High-throughput poly-sensitization samples**

| **Sample** | **Total IgE** | **Specific IgE** | **Allergen** | **Specific IgE** | **Allergen** |
|---|---|---|---|---|---|
| Sample 1 | 1044 | >100 | rBet V1 (birch) | 24.6 | rAra h2 (peanut) |
| Sample 2 | 626 | >100 | rFel d1 (cat) | 28.9 | rDer p2 (house dust mite) |

The invention may be defined by any of the following clauses:

### Clauses

1. A method for producing non-human conditionally immortalized mast cell progenitors, the method comprising:
   - introducing a nucleic acid molecule comprising an inducible homeobox gene into myeloid progenitor cells, wherein said myeloid progenitor cells are derived from a non-human animal and are engineered to express a heterologous high-affinity IgE receptor alpha subunit (FcεRIα);
   - selecting for cells which contain the nucleic acid molecule.
2. The method of clause 1, wherein the homeobox gene is selected from HoxB8, HoxA9, Lhx2 (LH2) and TLX1 (Hox11).
3. The method of clause 2, wherein the homeobox gene is HoxB8.
4. The method of any one of clauses 1 to 3, wherein the nucleic acid molecule further comprises a gene conferring resistance to an antibiotic, and wherein selecting for cells which contain the nucleic acid molecule comprises culturing the cells in a culture medium comprising the antibiotic.
5. The method of clause 4, wherein expression of the homeobox gene is controlled by an inducer, and wherein the culture medium further comprises the inducer.
6. The method of clause 5, wherein the inducer is 4-hydroxytamoxifen (4-OHT).
7. The method of any one of clauses 4 to 7, wherein the culture medium further comprises interleukin-3 (IL-3).
8. The method of any preceding clause, wherein the nucleic acid molecule is a vector, optionally a viral vector.
9. The method of clause 8, wherein the viral vector is a lentiviral particle.
10. The method of any preceding clause, further comprising carrying out a single cell dilution of the conditionally immortalized mast cell progenitors, followed by clonal expansion so as to obtain a monoclonal conditionally immortalized mast cell progenitor cell line.
11. The method of any preceding clause, further comprising deriving the myeloid progenitor cells from a non-human animal by:
   - providing whole bone marrow previously obtained from the animal;
   - enriching the bone marrow for hematopoietic progenitor cells, optionally using magnetic cell separation; and
   - culturing the hematopoietic progenitor cells in the presence of IL-3, optionally wherein the cells are cultured in WEHI-3B cell-conditioned medium.
12. The method of any preceding clause, wherein the non-human animal is a rodent, optionally a mouse.
13. The method of any preceding clause, wherein the heterologous FcεRIα is human FcεRIα.
14. The method of clause 5, or any one of clauses 6 to 13 when dependent on clause 5, wherein the method further comprises culturing the non-human conditionally immortalized mast cell progenitors in the absence of the inducer and in the presence of IL-3 so as to obtain differentiated mast cells.
15. The method of clause 14, wherein the non-human conditionally immortalized mast cell progenitors are cultured for at least 5 days.
16. The method of clause 14 or clause 15, wherein the non-human conditionally immortalized mast cell progenitors are cultured in WEH3b cell-conditioned medium.
17. A method for preparing mast cells comprising culturing non-human conditionally immortalized mast cell progenitors which are engineered to express a heterologous high-affinity IgE receptor alpha subunit (FcεRIα), wherein the conditionally immortalized mast cell progenitors further comprise a homeobox gene, the expression of the homeobox gene being under the control of an inducer, and wherein the conditionally immortalized mast cell progenitors are cultured in the absence of the inducer and in the presence of IL-3.
18. The method of any one of clauses 14 to 17, wherein the mast cells are double positive for c-kit and heterologous FcεRIα.
19. A non-human conditionally immortalized mast cell progenitor comprising a homeobox gene, the expression of the homeobox gene being under the control of an inducer, wherein said cell expresses a heterologous high-affinity IgE receptor alpha subunit (FcεRIα).
20. The cell of clause 19, wherein the cell is obtainable by the method of any one of clauses 1 to 14.
21. A composition comprising:
   - a population of non-human conditionally immortalized mast cell progenitors according to clause 19 or clause 20; and
   - the inducer.
22. A non-human mast cell, wherein the mast cell comprises a homeobox gene, the expression of the homeobox gene being under the control of an inducer, and wherein the mast cell expresses a heterologous high-affinity IgE receptor alpha subunit (FcεRIα).
23. The non-human mast cell of clause 22, wherein the mast cell is obtainable by the method of any one of clauses 14 to 18.
24. The non-human mast cell of clause 22 or clause 23, wherein the mast cell is c-kit positive.
25. A composition comprising a population of non-human mast cells according to any of clauses 22 to 24.
26. The composition of clause 25, wherein at least 95% of the mast cell population is c-kit positive.
27. The composition of clause 25 or clause 26, wherein the mast cell population displays from 15000-20000 FcεRIα receptors per cell, in the absence of IgE sensitization.
28. The composition of any of clauses 25 to 27, wherein the mast cell population displays from 80000 to 110000 FcεRIα receptors per cell, in the presence of IgE sensitization.
29. The composition of any of clauses 25 to 28, wherein the mast cells display a maximal activation of at least 90%, optionally at least 95%.
30. The composition of any of clauses 25 to 29, wherein the mast cells have a doubling time of less than 35 hours, optionally less than 30 hours.
31. A method for determining whether a patient is allergic to an allergen and/or the severity of a patient's allergy to an allergen, the method comprising:
   - incubating mast cells with a sample comprising patient antibodies;
   - contacting the mast cells with the allergen; and
   - detecting activation of the mast cells,
   wherein the mast cells are non-human mast cells according to clauses 22-24, or a population of non-human mast cells comprised within a composition according to clauses 25-30.
32. The method of clause 31, wherein the sample is neat or diluted patient serum.
33. The method of clause 31, wherein the sample comprises antibodies which have been isolated from patient serum, e.g. in a suitable medium or buffer.
34. The method of any one of clauses 31 to 33, wherein the mast cells are incubated with the sample comprising patient antibodies for at least 10 hours, preferably at least 12 hours (e.g. overnight).
35. The method of any one of clauses 31-34, wherein the method is carried out in the absence of a wash step between the steps of incubating the mast cells with the sample comprising patient antibodies and contacting the mast cells with the allergen.
36. The method of any one of clauses 31-35, wherein detecting activation of the mast cells comprises detecting the release of a mediator, detecting the expression of a surface marker, or detecting a pH change that is indicative of the presence of IgE specific for the allergen in the patient serum sample.
37. The method of clause 36, wherein the surface marker is a lysozyme associated membrane glycoprotein (such as LAMP-1, LAMP-2 or LAMP-3), CD203c, CD63 or CD107a, optionally wherein the surface marker is CD107a.
38. The method of clause 37, wherein detecting the expression of the surface marker comprises contacting the mast cells with an antibody specific for the surface marker, and detecting antibodies bound to the cells.
39. The method of clause 38, wherein the method comprises quantifying the antibodies bound to the cells, optionally using flow cytometry.
40. The method of clause 36, wherein the mediator is β-hexosaminidase, a protease, histamine or a leukotriene (e.g. LTC4).
41. The method of any one of clauses 31 to 40, wherein detecting activation of the mast cells further comprises determining a level of activation, the level of activation corresponding to the severity of the allergy of the patient to the allergen.
42. The method of any one of clauses 31 to 41, wherein the method is for determining whether the patient is allergic to multiple allergens, the method comprising:
   - separately incubating each of a plurality of mast cell populations with a sample comprising patient antibodies;
   - labelling each of the plurality of mast cell populations with a different detectable label;
   - after incubating the mast cell populations with the samples comprising patient antibodies, separately contacting each of the plurality of mast cell populations with a different allergen;
   - pooling the plurality of mast cell populations; and
   - detecting activation of the mast cells in each population,
   wherein each mast cell population comprises non-human mast cells according to any of clauses 22 to 24.
43. The method of clause 42, wherein the step of labelling each of the plurality of mast cell populations with a different detectable label is carried out before or after the step of incubating each of the plurality of mast cell populations with the samples comprising patient antibodies.
44. The method of any one of clauses 31 to 41, wherein the method is for determining whether multiple patients are allergic to an allergen, the method comprising:
   - separately incubating each of a plurality of samples, each sample comprising antibodies from a different patient, with one of a plurality of mast cell populations;
   - labelling each of the plurality of mast cell populations with a different detectable label;
   - pooling the plurality of mast cell populations;
   - contacting each of the plurality of mast cell populations with the allergen; and
   - simultaneously detecting activation of the mast cells in each population,
   wherein each mast cell population comprises non-human mast cells according to any of clauses 22 to 24.
45. The method of clause 44, wherein the step of labelling each of the plurality of mast cell population with a different detectable label is carried out before or after the step of incubating the mast cell populations with the samples comprising patient antibodies.
46. The method of clause 44 or clause 45, wherein the plurality of mast cell populations are pooled prior to contacting the mast cells with the allergen.
47. The method of clause 44 or clause 45, wherein the plurality of mast cell populations are pooled after contacting the mast cells with the allergen, and before detecting activation.
48. The method of any one of clauses 44 to 47, wherein the detectable label is a fluorescent dye.
49. A method for monitoring the effectiveness of a therapy that is being used, that may be used in the future, or that has previously been used to treat a patient allergic to an allergen, the method comprising:
   - incubating mast cells with a first sample comprising patient antibodies;
   - contacting the mast cells with the allergen;
   - determining a first level of activation of the mast cells; and
   - comparing the determined first level with a reference level,
   wherein the mast cells are non-human mast cells according to any of clauses 22 to 24.
50. The method of clause 49, wherein the reference level is a baseline level of mast cell activation determined using a sample comprising antibodies which were obtained from the patient prior to initiation of therapy.
51. The method of clause 49 or clause 50, wherein the therapy is allergen-specific immunotherapy (AIT).
52. The method of clause 51, wherein the patient is treated with AIT for at least 6 months, or at least 1, 2, 3, 4 or 5 years.
53. The method of clause 51 or clause 52, wherein the method is carried out at regular time intervals following initiation of AIT, optionally when the method is carried out approximately every 3, 4, 6 or 12 months.
54. The method of any one of clauses 49 to 53, wherein the method is for identifying patients who are non-responsive to the therapy.
55. The method of any one of clauses 49 to 53, wherein the method is for identifying patients who become tolerant to the allergen.
56. The method of clause 49 or clause 50, wherein the method is for monitoring the effectiveness of a therapy which comprises treatment of the patient with an anti-allergy therapeutic agent.
57. The method of clause 56, wherein the anti-allergy therapeutic agent is an anti-lgE agent, optionally wherein the anti-allergy therapeutic agent is an antibody, a DARPin, an affimer, a monobody, an anticalin or an affibody.
58. The method of clause 56, wherein the anti-allergy therapeutic agent is an agent which induces protective IgG.
59. The method of any one of clauses 49 to 58, the method further comprising detecting the induction of protective (antigen-specific) IgG by:
   - incubating mast cells with a second sample comprising patient antibodies, wherein the second sample is IgG-depleted;
   - contacting the mast cells with the allergen;
   - determining a second level of activation of the mast cells; and
   - comparing the determined second level with the reference level and/or with the determined first level.
60. A method for determining the potency of an allergen preparation, the method comprising:
   - incubating mast cells with IgE specific for the allergen;
   - contacting the mast cells with a sample of the allergen preparation;
   - determining a level of activation of the mast cells; and
   - optionally, comparing the determined level of activation with a reference level,
   wherein the mast cells are non-human mast cells according to any of clauses 22 to 24.
61. The method of clause 60, wherein the method is for detecting variations in potency between different batches of the same allergen preparation.
62. The method of clause 60 or clause 61, wherein the method is for preparing a standardised allergen preparation, and the method further comprises adjusting the potency of the allergen preparation.
63. A method for allergenicity screening of a food additive or drug candidate, the method comprising:
   - incubating mast cells with a sample comprising subject antibodies;
   - contacting the mast cells with the food additive or drug candidate; and
   - detecting activation of the mast cells,
   wherein the mast cells are non-human mast cells according to any of clauses 22 to 24.
64. The method of clause 63, wherein the sample comprises antibodies obtained from a plurality of individuals, e.g. at least 10, 50, 100, 200, 500, 700 or 1000 individuals.
65. A method for determining the serum IgE concentration of a patient, the method comprising:
   - incubating mast cells with a sample comprising IgE from the patient; and
   - determining the amount of IgE bound to the surface of the mast cells,
   wherein the mast cells are non-human mast cells according to any of clauses 22 to 24.
66. The method of clause 65, wherein the method is for determining the total IgE concentration in the sample.
67. The method of clause 65, wherein the method is for determining the concentration of an allergen-specific IgE in the sample.
68. The method of any one of clauses 65 to 67, wherein determining the amount of IgE bound to the surface of the mast cells comprises:
   - contacting the mast cells with an agent that specifically binds to the IgE; and
   - determining the amount of the agent bound to the cells.
69. The method of clause 68, wherein the method further comprises comparing the amount of agent bound to the cells with a reference.
70. The method of clause 69, wherein the reference is a standard curve.
71. The method of any one of clauses 68 to 70, wherein the agent is an anti-lgE antibody or the cognate allergen of the allergen-specific IgE.
72. The method of any one of clauses 68 to 71, wherein the agent is labelled with a detectable label, e.g. a fluorescent dye.
73. A kit for allergy testing, the kit comprising:
   - non-human mast cells according to any of clauses 22 to 24;
   - a reagent for detecting activation of the mast cells;
   - optionally, one or more allergens; and
   - optionally, a positive control (e.g. monoclonal IgE which is specific for the or each allergen).
74. The kit of clause 73, wherein the reagent is an antibody (e.g. an antibody specific for a lysozyme associated membrane glycoprotein (LAMP), such as CD107a or CD203c), a protease substrate (e.g a tryptase substrate), a substrate of beta-hexosaminidase or a reagent that is responsive to changes in pH (e.g. pH-sensitive fluorophores or pH indicator solutions).
75. The method of any one of clauses 1 to 17 or 31 to 72, the cells of any one of clauses 19, 20, or 22 to 24, the composition of any one of clauses 21 or 25 to 30, or the kit of any one of clauses 73 to 74, wherein the patient is human and the FcεRIα is human FcεRIα (huFcεRIα).

## Claims

1. A method for producing non-human conditionally immortalized mast cell progenitors, the method comprising:
- introducing a nucleic acid molecule comprising an inducible homeobox gene into myeloid progenitor cells, wherein said myeloid progenitor cells are derived from a non-human animal and are engineered to express a heterologous high-affinity IgE receptor alpha subunit (FcεRIα); and
- selecting for cells which contain the nucleic acid molecule.

2. The method of claim 1, wherein the homeobox gene is selected from HoxB8, HoxA9, Lhx2 (LH2) and TLX1 (Hox11), optionally wherein the homeobox gene is HoxB8.

3. The method of claim 1 or claim 2, wherein:
the nucleic acid molecule further comprises a gene conferring resistance to an antibiotic, and wherein selecting for cells which contain the nucleic acid molecule comprises culturing the cells in a culture medium comprising the antibiotic; and
expression of the homeobox gene is controlled by an inducer, and wherein the culture medium further comprises the inducer and interleukin-3 (IL-3), optionally wherein the inducer is 4-hydroxytamoxifen (4-OHT).

4. The method of any preceding claim, further comprising deriving the myeloid progenitor cells from a non-human animal by:
- providing whole bone marrow previously obtained from the animal;
- enriching the bone marrow for hematopoietic progenitor cells, optionally using magnetic cell separation; and
- culturing the hematopoietic progenitor cells in the presence of IL-3, optionally wherein the cells are cultured in WEHI-3B cell-conditioned medium,
optionally wherein the non-human animal is a rodent, such as a mouse.

5. The method of claim 3, or claim 4 when dependent on claim 3, wherein the method further comprises culturing the non-human conditionally immortalized mast cell progenitors in the absence of the inducer and in the presence of IL-3 so as to obtain differentiated mast cells, optionally wherein the non-human conditionally immortalized mast cell progenitors are cultured for at least 5 days.

6. A non-human conditionally immortalized mast cell progenitor comprising a homeobox gene, the expression of the homeobox gene being under the control of an inducer, wherein said progenitor expresses a heterologous high-affinity IgE receptor alpha subunit (FcεRIα).

7. A non-human mast cell, wherein the mast cell comprises a homeobox gene, the expression of the homeobox gene being under the control of an inducer, wherein the mast cell expresses a heterologous high-affinity IgE receptor alpha subunit (FcεRIα), and wherein the mast cell is c-kit positive.

8. A method for determining whether a patient is allergic to an allergen and/or the severity of a patient's allergy to an allergen, the method comprising:
- incubating mast cells with a sample comprising patient antibodies;
- contacting the mast cells with the allergen; and
- detecting activation of the mast cells,
wherein the mast cells are non-human mast cells according to claim 7.

9. The method of claim 8, wherein detecting activation of the mast cells comprises detecting the release of a mediator, detecting the expression of a surface marker, or detecting a pH change that is indicative of the presence of IgE specific for the allergen in the patient serum sample, optionally wherein the surface marker is a lysozyme associated membrane glycoprotein (such as LAMP-1, LAMP-2 or LAMP-3), CD203c, CD63 or CD107a, preferably wherein the surface marker is CD107a.

10. A method for monitoring the effectiveness of a therapy that is being used, that may be used in the future, or that has previously been used to treat a patient allergic to an allergen, the method comprising:
- incubating mast cells with a first sample comprising patient antibodies;
- contacting the mast cells with the allergen;
- determining a first level of activation of the mast cells; and
- comparing the determined first level with a reference level,
wherein the mast cells are non-human mast cells according to claim 10,
optionally wherein the reference level is a baseline level of mast cell activation determined using a sample comprising antibodies which were obtained from the patient prior to initiation of therapy,
optionally wherein the therapy is allergen-specific immunotherapy (AIT).

11. A method for determining the potency of an allergen preparation, the method comprising:
- incubating mast cells with IgE specific for the allergen;
- contacting the mast cells with a sample of the allergen preparation;
- determining a level of activation of the mast cell; and
- optionally, comparing the determined level of activation with a reference level,
wherein the mast cells are non-human mast cells according to claim 10.

12. A method for allergenicity screening of a food additive or drug candidate, the method comprising:
- incubating mast cells with a sample comprising subject antibodies;
- contacting the mast cells with the food additive or drug candidate; and
- detecting activation of the mast cells,
wherein the mast cells are non-human mast cells according to claim 10.

13. The method of any one of claims 8 to12, wherein the method is carried out in the absence of a wash step between the steps of incubating the mast cells with the sample comprising patient antibodies, or with the IgE specific for the allergen, and contacting the mast cells with the allergen, allergen preparation, food additive or drug candidate.

14. A method for determining the serum IgE concentration of a patient, the method comprising:
- incubating mast cells with a sample comprising IgE from the patient; and
- determining the amount of IgE bound to the surface of the mast cells,
wherein the mast cells are non-human mast cells according to claim 10.

15. The method of any one of claims 1 to 5 or 8 to 14, or the cells of claim 6 or claim 7, wherein the FcεRIα is human FcεRIα (huFcεRIα), optionally wherein the patient is human.
